# EUROPEAN PATENT SPECIFICATION

(11) **EP 4 591 921 B1**
(45) Date of publication and mention of the grant of the patent: **05.08.2026**
(21) Application number: 25153357.6
(22) Date of filing: 22.01.2025
(51) Int. Cl.: A61N 1/36, G16H 20/30, A61N 1/372

(54) **RECHARGE INTERVAL FOR MEDICAL DEVICE**
WIEDERAUFLADUNGSINTERVALL FÜR EINE MEDIZINISCHE VORRICHTUNG
INTERVALLE DE RECHARGE POUR DISPOSITIF MÉDICAL

(30) Priority: 26.01.2024 US 202463625808 P; 20.12.2024 US 202418990837
(43) Date of publication of application: 30.07.2025
(73) Proprietor: Medtronic, Inc., Minneapolis, MN 55432 (US)
(72) Inventor: Klotsche, Emily G., Mounds View, MN, 55112 (US); Georgen, Christopher M., Minneapolis, MN, 55432 (US); Lee, Mathew J., Minneapolis, MN, 55432-3568 (US); Fried, Andrew T., Minneapolis, MN, 55432-3568 (US); Alberti, Laszlo S., Minneapolis, MN, 55432-3568 (US); Paralikar, Kunal J., Minneapolis, MN, 55432-3568 (US)
(74) Representative: Zimmermann & Partner Patentanwälte mbB

(56) References cited:
- WO-A1-2018/187734
- US-A1- 2010 114 215
- US-A1- 2010 114 252
- US-A1- 2011 106 213

## Description

This application claims the benefit of U.S. Provisional Application No. 63/625,808, filed January 26, 2024, and entitled, "RECHARGE INTERVAL FOR MEDICAL DEVICE,".

### TECHNICAL FIELD

The disclosure relates to implantable medical devices, and, more specifically, rechargeable implantable medical devices.

### BACKGROUND

Medical devices may be external or implanted and may be used to monitor patient signals such as cardiac activity, biological impedance and to deliver electrical stimulation therapy to patients via various tissue sites to treat a variety of symptoms or conditions such as chronic pain, tremor, Parkinson's disease, epilepsy, urinary or fecal incontinence, sexual dysfunction, obesity, or gastroparesis and other conditions. In some examples, medical devices may include a rechargeable electrical power source, or may be powered directly by transmitting energy through tissue. Documents US 2010/114252 A1 and US 2010/114215 A1 relate to rechargeable medical devices.

### SUMMARY

The invention is defined by the appended claims. In general, the disclosure is directed to devices, systems, and techniques for determining and outputting, for display to a user, suggested recharge intervals (and/or suggested recharge frequencies) for a rechargeable implantable medical device (IMD). More particularly, this disclosure is directed to devices, systems, and techniques for determining suggested recharge interval values that may facilitate regular charging schedules and/or charging habits and enable a user (e.g., a patient) to more easily remember when to recharge the IMD. The suggested recharge intervals may align with time intervals that would normally occur on a patient's schedule and/or are otherwise easier for a user to remember.

In examples described herein, a system is configured to determine one or more suggested recharge interval values for one or more therapy programs based on information about a power source of an IMD. The suggested recharge interval values may be a values that are relevant to the battery depletion rate of the IMD and also easy for a user (e.g., a patient, clinician, caregiver, etc.) to remember which may align with time intervals that would normally occur on a user's schedule or are otherwise easier for compliance. In particular, these suggested recharge interval values may be beneficial for IMDs that require relatively infrequent recharging on the order of several days, weeks, months, years, or longer.

For example, instead of non-integer and/or irregular charging intervals (e.g., 16.5 days, 34.5 days, 7 weeks), the systems described herein may be configured to determine and generate integer values and/or regular charging intervals (e.g., 2 weeks, 1 month, 2 months, 6 months, 1 year, every full moon, every four full moons, and/or the like), which may facilitate less skipped or missed charging sessions due to users forgetting about charging due to irregular and relatively long charging intervals. In some examples, the suggested recharge intervals described in this disclosure may be shorter and/or more frequent than otherwise needed (e.g., because the power source of the IMD does not actually need to be recharged), however the suggested recharge intervals discussed herein may nonetheless lead to more effective therapy, reduced programming time (e.g., because the determination and presentation of suggested recharge intervals automatically occurs by the system), reduced human error, and reduced mental burden on patients, clinicians, and/or other caregivers. Further, power sources of IMDs may have increased longevity because of more consistent charging habits that may result by implementing the techniques of this disclosure. Lastly, the increase in compliance related to recharging can reduce patient risks related to IMD battery depletion and loss of therapy, which for some neuromodulation therapies could have severe consequences.

In examples described herein, the system uses information from therapy programming (e.g., therapy parameters and/or therapy schedules), as well as IMD information (e.g., information about the power source of the IMD or information about therapy delivered) to determine one or more suggested recharge intervals. In some examples, the system can receive information to determine one or more recharge interval values, which indicates a period of time after which a power source of the IMD will run out of charge and/or drop below a predetermined level. The suggested recharge intervals determined by the system may be different from, but based on, the determined recharge intervals. For example, the system may determine a suggested recharge interval value from multiple possible predefined possible recharge interval values. In some examples, the system rounds a determined (e.g., calculated) recharge interval value to one of a plurality of possible suggested recharge interval values. In some examples, the predefined possible suggested recharge interval values correspond to different respective ranges of possible values of the determined recharge interval value. In some examples, bins correlate ranges of the possible values of the determined recharge interval value to the plurality of predefined possible suggested recharge interval values (e.g., for a given therapy schedule and/or a given set of therapy parameters).

In some examples, the system determines multiple suggested recharge interval values, such as for different therapy schedules that the patient may use during overall therapy. In some examples, the system is configured to update suggested recharge intervals, e.g., based on updated therapy parameters, a transition between therapy schedules, and/or based on records of therapy actually delivered.

In some examples, the suggested recharge interval values and/or the determined (e.g., calculated) recharge interval values may be generated for output to a user, e.g., on a user interface. For example, a user interface of a programmer, an external charging device, and/or another device, may be configured to display the suggested recharge interval values. The system may be configured to generate prompts or other messages related to recharging, such as prompts to recharge based on the suggested recharge interval values.

In one example, a system includes processing circuitry configured to receive information of a therapy program for an implantable medical device. The information includes therapy parameters values and a therapy schedule defining delivery of therapy by the implantable medical device. The processing circuitry is configured to determine, based on the therapy parameter values and the therapy schedule of the therapy program, a recharge interval value for a power source of the implantable medical device. The processing circuitry is configured to determine, for the therapy program, a suggested recharge interval value from a plurality of predefined possible suggested recharge interval values corresponding to different respective ranges of possible values of the determined recharge interval value. The processing circuitry is configured to generate, for output to a user and for the therapy program, an indication of the suggested recharge interval value.

In another example, a method includes receiving, by processing circuitry, information of a therapy program for an implantable medical device. The information includes therapy parameters values and a therapy schedule defining delivery of therapy by the implantable medical device. The method includes determining, by the processing circuitry and based on the therapy parameter values and the therapy schedule of the therapy program, a recharge interval value for a power source of the implantable medical device. The method includes determining, by the processing circuitry and for the therapy program, a suggested recharge interval value from a plurality of predefined possible suggested recharge interval values corresponding to different respective ranges of possible values of the determined recharge interval value. The method includes generating for output to a user, by the processing circuitry and for the therapy program, an indication of the suggested recharge interval value.

In another example, a system includes processing circuitry configured to receive information of a therapy program for an implantable medical device. The information includes therapy parameters values and a therapy schedule defining delivery of therapy by the implantable medical device. The processing circuitry is configured to determine, based on the therapy parameter values and the therapy schedule of the therapy program, a recharge interval value for a power source of the implantable medical device. The processing circuitry is configured to compare the determined recharge interval value to a range of the possible values of the determined recharge interval value for each bin of a plurality of bins, wherein each bin of the plurality of bins correlates ranges of the possible values of the determined recharge interval value to a plurality of predefined possible suggested recharge interval values. The processing circuitry is configured to, based on the comparison, select the suggested recharge interval value from the plurality of predefined possible suggested recharge interval values that corresponds to the bin in which the determined recharge interval value resides. The processing circuitry is configured to generate, for output to a user and for the therapy program, an indication of the suggested recharge interval value, wherein the output includes a prompt on a user interface of a computing device indicating the suggested recharge interval value.

In another example, a computer-readable medium includes instructions that, when executed, control processing circuitry to receive information of a therapy program for an implantable medical device. The information includes therapy parameters values and a therapy schedule defining delivery of therapy by the implantable medical device. The computer-readable medium includes instructions that, when executed, control processing circuitry to determine, based on the therapy parameter values and the therapy schedule of the therapy program, a recharge interval value for a power source of the implantable medical device. The computer-readable medium includes instructions that, when executed, control processing circuitry to determine, for the therapy program, a suggested recharge interval value from a plurality of predefined possible suggested recharge interval values corresponding to different respective ranges of possible values of the determined recharge interval value. The computer-readable medium includes instructions that, when executed, control processing circuitry to generate, for output to a user and for the therapy program, an indication of the suggested recharge interval value.

An example system includes processing circuitry configured to receive information of a therapy program for an implantable medical device. The information of the therapy program includes therapy parameters values and a therapy schedule defining delivery of therapy by the implantable medical device. The processing circuitry is configured to determine, based on the therapy parameter values and the therapy schedule of the therapy program, a recharge interval value for a power source of the implantable medical device. The processing circuitry is configured to determine, for the therapy program, a suggested recharge interval value from a plurality of predefined possible suggested recharge interval values corresponding to different respective ranges of possible values of the determined recharge interval value. The processing circuitry is configured to generate, for output to a user and for the therapy program, an indication of the suggested recharge interval value. The details of one or more examples of the disclosure are set forth in the accompanying drawings and the description below. Other features, objects, and advantages of the disclosure will be apparent from the description and drawings, and from the claims.

### BRIEF DESCRIPTION OF DRAWINGS

FIG. 1 is a conceptual diagram illustrating an example medical device system including an including an implantable medical device, which may be a leadless neurostimulation device, implanted near a tibial nerve in a leg of a patient.
FIG. 2 is a block diagram illustrating example components of the implantable medical device of FIG. 1.
FIG. 3 is a block diagram of an example external charging device.
FIG. 4 is a block diagram of an example programmer.
FIG. 5 is a table showing example suggested recharge intervals based on determined (e.g., calculated) recharge intervals.
FIGS. 6A, 6B, 6C, 6D, 6E, 6F, and 6G are conceptual diagrams illustrating example user interfaces related to suggested recharge intervals according to this disclosure.
FIG. 7 is a flow chart illustrating an example technique for determining and generating recharging information.
FIG. 8 is a flow chart illustrating an example technique for determining and generating recharging information.
FIG. 9 is a flow chart illustrating an example technique for determining and generating recharging information.

Like reference characters denote like elements throughout the description and figures.

### DETAILED DESCRIPTION

This disclosure describes devices, systems, and techniques for determining and generating for output, to a user, suggested recharge intervals for a rechargeable power source of implantable medical device (IMD). Some medical devices systems generate information about when a user should recharge the power source of the IMD. In some medical device systems that are programmed according to known (e.g., predetermined) schedules defining instances of therapy delivery (i.e., a therapy schedule), some medical devices systems can generate a recharging interval and/or recharging frequency based on the therapy schedule to ensure that the power source of the IMD has enough energy to deliver therapy according to the therapy schedule. These systems can calculate the recharge interval based on physical properties of the power source (e.g., the power source capacity), as well as predicted usage that causes drain on the power source. In some examples, such as examples with infrequent, low-power therapy (e.g., such as in examples of therapy for incontinence where stimulation is delivered to the tibial nerve), the recharging interval may include relatively long durations of time between charging sessions, meaning only relatively infrequent charging is needed. However, the suggested recharge interval generated by some medical device systems are often non-integer and/or irregular charging interval (e.g., 16.5 days, 34.5 days, 7 weeks), which may be difficult for a user (e.g., a patient in which the IMD is implanted and/or a caretaker) to remember, and may also cause increased mental burden when a user plans for recharging. For example, the user may need to remember to keep checking for battery charge status during these long intervals, but the user may forget to check battery status or recharge the IMD when many days, weeks, and/or months pass. These non-integer and/or irregular charging intervals (which may be difficult to remember) may lead to skipped recharging sessions and/or skipped therapy sessions because the power source of the medical device was not charged according to the suggested recharge interval. This may, in turn, lead to less effective therapy.

As described herein, a system may be configured to determine one or more suggested recharge interval values for one or more therapy programs based on information about a power source of an IMD and based on predicted future use according to one or more therapy schedules. The suggested recharge interval values may be a values that are easier for a user (e.g., a patient, clinician, caregiver, etc.) to remember and may align with time intervals that would normally occur on a user's schedule. For example, the systems described herein may be configured to determine and generate suggested recharge interval values that include integer values and/or regular charging interval (e.g., 2 weeks, 1 month, 2 months, every full moon, and/or the like). These suggested recharge interval values that include integer values and/or regular charging interval values may enable a user (e.g., a patient) to more easily remember when to recharge, especially as compared to non-integer and/or irregular charging intervals. These suggested recharge intervals determined by the systems described herein may facilitate more regular recharging and/or more regular charging habits. These suggested recharge intervals may facilitate less skipped or missed charging sessions due to users forgetting about charging because of irregular charging schedules. In some examples, the suggested recharge intervals described in this disclosure may be shorter and/or more frequent than otherwise needed (e.g., because the power source of the IMD does not actually need to be recharged). By determining more regular and/or easier to remember suggested recharge intervals, the techniques of this disclosure may facilitate more effective therapy, reduced programming time (e.g., because the determination and presentation of suggested recharge intervals is performed automatically by the medical device system), reduced human error, and reduced mental burden on patients, clinicians, and/or other caregivers. Further, power sources of IMDs may have increased longevity because of more consistent charging habits that may result by implementing the techniques of this disclosure.

In examples described herein, an IMD includes a rechargeable power source (e.g., a battery), and is configured to deliver therapy according to one or more therapy schedules. In some examples, one or more components of the system includes suitable hardware and/or software configurations for determining charging and/or recharging information about the rechargeable power source (e.g., components for measuring or estimating energy levels and/or energy consumption of the rechargeable power source). Information of the power source may be used for determination of the suggested charging intervals according to the techniques described herein.

In examples described herein, the system uses information from therapy programming (e.g., therapy parameters and/or therapy schedules), as well as IMD information, including from the power source of the IMD, to determine one or more suggested recharge intervals. In some examples, the system can receive information to determine one or more determined (e.g., calculated) recharge interval values, which may indicate a period of time a power source of the IMD to run out of charge and/or drop below a predetermined level. The suggested recharge intervals determined by the system may be different from, but based on, the determined (e.g., calculated) recharge intervals. For example, the system may determine a suggested recharge interval value from multiple possible predefined possible recharge interval values correlating to recharge interval values. In some examples, the system rounds a determined (e.g., calculated) recharge interval value to one of a plurality of possible suggested recharge interval values. In some examples, the predefined possible recharge interval values are based on a plurality of bins that are defined in terms of possible values of the recharge interval value, such that the system selects the bin corresponding to the recharge interval value for a given therapy program (e.g., for a given therapy schedule and/or a given set of therapy parameters). In this manner, these bins may be an example of different ranges of recharge interval values that may correspond to different respective different predefined possible recharge interval values.

In some examples, the system determines multiple suggested recharge interval values, such as for different therapy schedules. In some examples, the system is configured to update suggested recharge intervals, e.g., based on updated therapy parameters, a transition between therapy schedules, and/or based on records of therapy actually delivered.

In some examples, the suggested recharge interval values may be generated for output to a user, e.g., on a user interface. For example, a user interface of a programmer, or another device, may be configured to display the suggested recharge interval values. The system may be configured to generate prompts or other messages related to recharging, such as prompts to recharge based on the suggested recharge interval values.

Although this disclosure is discussed primarily in terms of intervals of time (e.g. interval values), which may represent a length or period of time (e.g., 1 week), it should be understood that frequencies (e.g., frequency values), which represent a recurring event (e.g., every day, every week, once per week, weekly, etc.) are contemplated to be used in addition to or instead of intervals, such as in any examples discussed herein where a system is configured to determine, use, and/or display such values in the context of recharging a power source of an IMD.

Additionally, although the devices, systems, and techniques described herein are described primarily in the context of a rechargeable power source (e.g., a rechargeable battery) of IMDs for a tibial nerve stimulator configured to provide tibial nerve stimulation, the techniques described herein may be applicable to other devices configured for other types of therapy. For example, the techniques of this disclosure may be applicable for other types of devices configured for invasive or noninvasive neuromodulation for pain relief, muscle activation, and/or other therapeutic benefits such as, but not limited to, deep brain stimulation (DBS), spinal cord stimulation (SCS), sacral nerve stimulation (SNS), cardiac stimulation, pacing, defibrillation, or other cardiac therapy, peripheral nerve stimulation or therapy, drug delivery (e.g., via a drug pump), circulatory support (e.g., mechanical circulatory support), or any other device (e.g., medical device) that includes a power source. Additionally, the techniques of this disclosure are not limited to rechargeable power sources, but are also applicable to other types of power sources (e.g., non-rechargeable power sources, primary cell, etc.), such as in instances a power source needs to be replaced or otherwise will cause a device to not operate as intended.

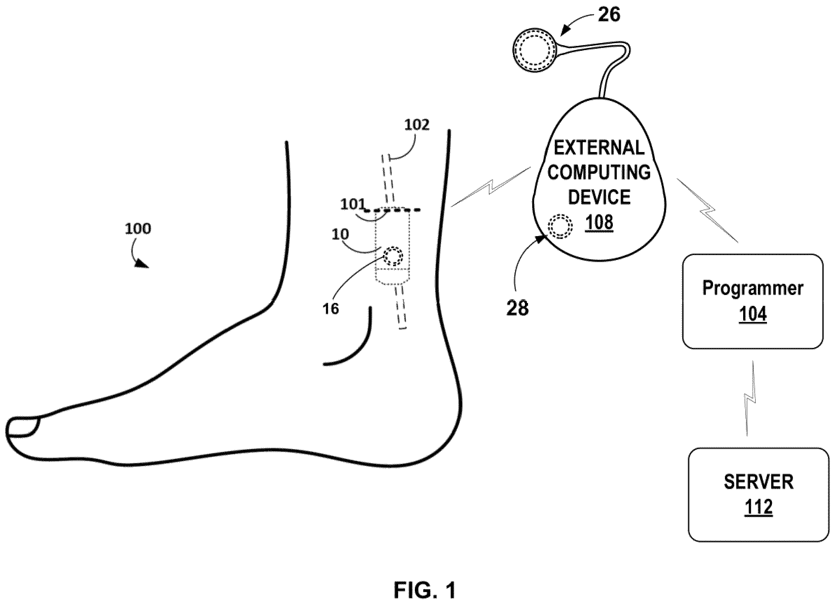

FIG. 1 is a conceptual diagram illustrating an example system that includes an implantable medical device and an external charging device that charges a rechargeable power source. FIG. 1 includes a system 100 including an implantable medical device (IMD) 10, an external computing device 108, a programmer 104 (which may be a patient programmer or a clinician programmer), and a server 112. In other examples, the techniques of this disclosure may be implemented in other battery powered devices, for example, an implantable drug pump.

External computing device 108 includes one or more charging coils, such as external primary coil 26 or internal primary coil 28. External computing device 108 may be used to program or adjust settings of IMD 10 and may also recharge an electrical energy storage device, such as a battery, of IMD 10. External computing device 108 may also communicate with server 112. In other examples, an external device (e.g., programmer 104) separate from external computing device 108 may communicate with IMD 10 to adjust therapy and/or sensing parameters, download recorded data, or perform other functions.

Server 112 may be one or more servers in a local network or in a cloud computing environment. Server 112 may be configured to communicate with programmer 104, external computing device 108 and/or IMD 10 via wireless communication through a network access point (not shown in FIG. 1) and may be co-located with external computing device 108 and/or programmer 104, or may be located elsewhere, such as in a cloud computing data center.

The example of FIG. 1 is a side view of a patient's leg showing a leadless neurostimulation IMD 10 near the ankle adjacent to a tibial nerve 102. IMD 10 can be implanted through the patient's skin and cutaneous fat layer via a small incision 101 (e.g., about one to three centimeters (cm)) above the tibial nerve on a medial aspect of the patient's ankle. While incision 101 is shown approximately horizontal to the length of the tibial nerve, other incisions or implantation techniques could be used according to physician preference. The example of FIG. 1 describes a neurostimulation implantable medical device for tibial nerve stimulation. In other examples, the techniques of this disclosure may apply to other devices, such as implantable neurostimulation system for use in spinal cord stimulation therapy and deep brain stimulation, as well as to other types of medical devices without limitation.

IMD 10 may be positioned adjacent to the region defined by flexor digitorum longus and soleus in which tibial nerve 102 is contained and implanted adjacent and proximal to a fascia layer. One or more electrodes of IMD 10 may face toward tibial nerve 102. Though not shown in FIG. 1, IMD 10 may also connect to one or more leads comprising one or more electrodes (not shown in FIG. 1).

IMD 10 may be constructed of any polymer, metal, or composite material sufficient to house the components of IMD 10. In some examples, IMD 10 is constructed with a biocompatible housing, such as titanium or stainless steel, or a polymeric material such as silicone or polyurethane, and surgically implanted at a site in patient near the tibial nerve. In other examples, IMD 10 is implanted near the pelvis, abdomen, or buttocks. The housing of IMD 10 may be configured to provide a hermetic seal for components, such as a rechargeable power source. In addition, the housing of IMD 10 may be selected of a material that facilitates receiving energy to charge the rechargeable power source.

Optional testing of neurostimulation IMD 10 may be performed to determine if IMD 10 has been properly positioned in proximity to tibial nerve 102 to elicit a desired response from an applied electrical stimulation. In an example, IMD 10 is controlled by programmer 104 or external computing device 108 to deliver test stimulation, and one or more indicative responses are monitored, such as toe flexion from simulation of the tibial motor neurons controlling the flexor hallucis brevis or flexor digitorum brevis, or a tingling sensation in the heel or sole of the foot excluding the medial arch. If such testing does not elicit appropriate motor or sensory responses, a clinician or other user may reposition IMD 10 and retest.

Once the clinician or other user has determined IMD 10 is properly positioned to provide an appropriate patient response to delivered stimulation therapy, the housing of device can be secured in place as needed. Securing IMD 10 may be optional as the natural shape of the region in which IMD 10 is implanted, and the shape of IMD 10 itself may have good compatibility with the surrounding tissue thus preventing IMD 10 from shifting or rolling after implantation. In some examples, leadless neurostimulation IMD 10 may further include one or more suture points to help secure IMD 10 to fascia or other parts of the patient. In some examples, a suture anchor may be included, such as at the distal end of the housing of IMD 10.

During operation, an electrical stimulation signal may be transmitted between one or more electrodes through the fascia layer. The electrical signal may be used to stimulate tibial nerve 102 which may be useful in the treatment of overactive bladder (OAB) symptoms of urinary urgency, urinary frequency and/or urge incontinence, fecal incontinence, pain or other symptoms.

In some examples, disease, age, and injury may impair physiological functions of a patient. In one example, bladder dysfunction, such as overactive bladder, urgency, or urinary incontinence, is a problem that may afflict people of all ages, genders, and races. Various muscles, nerves, organs, and conduits within the pelvic floor cooperate to collect, store and release urine. A variety of disorders may compromise urinary tract performance, and contribute to an overactive bladder, urgency, or urinary incontinence that interferes with normal physiological function. System 100 may help relieve some symptoms of some disorders.

Urinary incontinence may include urge incontinence and stress incontinence. In some examples, urge incontinence may be caused by disorders of peripheral or central nervous systems that control bladder micturition reflexes. Some patients may also suffer from nerve disorders that prevent proper triggering and operation of the bladder, sphincter muscles or nerve disorders that lead to overactive bladder activities or urge incontinence. In some cases, urinary incontinence may be attributed to improper sphincter function, either in the internal urinary sphincter or external urinary sphincter.

One type of therapy for treating bladder dysfunction includes delivery of electrical stimulation to a target tissue site within a patient to cause a therapeutic effect during delivery of the electrical stimulation. For example, delivery of electrical stimulation from IMD 10 to a target therapy site, e.g., a tissue site that delivers stimulation to modulate activity of a tibial nerve, spinal nerve (e.g., a sacral nerve), a pudendal nerve, dorsal genital nerve, an inferior rectal nerve, a perineal nerve, or branches of any of the aforementioned nerves, may provide a therapeutic effect for bladder dysfunction, such as a desired reduction in frequency of bladder contractions. In some cases, electrical stimulation of the tibial nerve may modulate afferent nerve activities to restore urinary function.

Bladder dysfunction generally refers to a condition of improper functioning of the bladder or urinary tract, and may include, for example, an overactive bladder, urgency, or urinary incontinence. Overactive bladder (OAB) is a patient condition that may include symptoms, such as urgency, with or without urinary incontinence. Urgency is a sudden, compelling urge to urinate, and may often, though not always, be associated with urinary incontinence. Urinary incontinence refers to a condition of involuntary loss of urine, and may include urge incontinence, stress incontinence, or both stress and urge incontinence, which may be referred to as mixed urinary incontinence. As used in this disclosure, the term "urinary incontinence" includes disorders in which urination occurs when not desired, such as stress or urge incontinence. Other bladder dysfunctions may include disorders such as non-obstructive urinary retention.

In some examples, the techniques described in this disclosure are directed to delivery of neurostimulation therapy in a non-continuous manner which may include on-cycles and off-cycles. For example, an IMD may deliver neurostimulation therapy for a specified period of time followed by a specified period of time when the IMD does not deliver neurostimulation (e.g., withholds delivery of neurostimulation). A period during which stimulation is delivered (an on-cycle) may include on and off periods (e.g., a duty cycle or bursts of pulses) with short inter-pulse durations of time when pulses are not delivered. In some examples, IMD 10 may switch between different operational modes that have different power consumptions for delivery of stimulation and non-delivery of stimulation in order to conserve power when stimulation is not to be delivered. In some examples, a continuous off period may be comparatively long, such as on the order of several days or even several weeks at a time.

The rechargeable power source of IMD 10 may include one or more capacitors, batteries, or other components (e.g., chemical or electrical energy storage devices). Example batteries may include lithium-based batteries, nickel metal-hydride batteries, or other materials. The rechargeable power source may be replenished, refilled, or otherwise capable of increasing the amount of energy stored after energy has been depleted. IMD 10 may include a secondary coil 16, wherein the energy received from secondary coil 16 may be conditioned and/or transformed by a charging circuit. The charging circuit may then send an electrical signal used to charge the rechargeable power source when the power source is fully depleted or only partially depleted.

External computing device 108 may be used to charge (e.g., recharge) the rechargeable power source within IMD 10 implanted in the patient. External computing device 108 may be a hand-held device, a portable device, or a stationary charging system. External computing device 108 may also be referred to as a charging device 108 or an external charging device 108 in this disclosure. External computing device 108 may include components necessary to charge IMD 10 through tissue of the patient. External computing device 108 may include an internal primary coil 28 and external primary coil 26. In other examples, external computing device may only include internal primary coil 28 and omit the use of external primary coil 26, or only include external primary coil 26 and omit the use of internal primary coil 28. External computing device 108 may include a housing to enclose operational components such as a processor, memory, user interface, telemetry module, power source, and charging circuit configured to transmit energy to secondary coil 16 via external primary coil 26 and/or internal primary coil 28. Although a user may control the recharging process with a user interface of external computing device 108, external computing device 108 may alternatively be controlled by another device, e.g., programmer 104, a computing device of server 112 such as a tablet computer, laptop or other similar computing device. The second external computing device of server 112 may include a computing device with a touch-screen user interface. In other examples, external computing device 108 may be integrated with an external programmer, such as patient programmer 104, which may be carried by the patient.

External computing device 108 and IMD 10 may utilize any wireless power transfer techniques that are capable of recharging the power source of IMD 10 when IMD 10 is implanted within the patient. In some examples, system 100 may utilize inductive coupling between internal primary coil 28 and/or external primary coil 26 of external computing device 108 and secondary coils (e.g., secondary coil 16) of IMD 10. In inductive coupling, internal primary coil 28 is placed near implanted IMD 10 such that internal primary coil 28 is aligned with secondary coil 16 of IMD 10. External computing device 108 may then generate an electrical current in internal primary coil 28 based on a selected power level for charging the rechargeable power source of IMD 10. When either internal primary coil 28 or external primary coil 26 are aligned with secondary coil 16, the electrical current in either internal primary coil 28 or external primary coil 26 may magnetically induce an electrical current in secondary coil 16 within IMD 10. Since secondary coil 16 is associated with and electrically coupled to the rechargeable power source, the induced electrical current may be used to increase the voltage, or charge level, of the rechargeable power source. Although inductive coupling is generally described herein, any type of wireless energy transfer may be used to transfer energy between external computing device 108 and IMD 10.

External primary coil 26 and/or internal primary coil 28 may include a wound wire (e.g., a coil) (not shown in FIG. 1). The coil may be constructed of a wire wound in an in-plane spiral (e.g., a disk-shaped coil). In some examples, this single or even multi-layers spiral of wire may be considered a flexible coil capable of deforming to conform with a non-planar skin surface. The coil may include wires that electrically couple the flexible coil to a power source and a charging module configured to generate an electrical current within the coil. Internal primary coil 28 may be external of the housing of external computing device 108 such that internal primary coil 28 can be placed on the skin of the patient proximal to IMD 10. In some examples, internal primary coil 28 may be disposed on the outside of the housing or even within housing.

Either external primary coil 26 and/or internal primary coil 28 of system 100 may include a heat sink device (not shown in FIG. 1). In the example of system 100, external computing device 108 is the power transmitting unit and IMD 10 is the power receiving unit. IMD 10 may be in a flipped or non-flipped position.

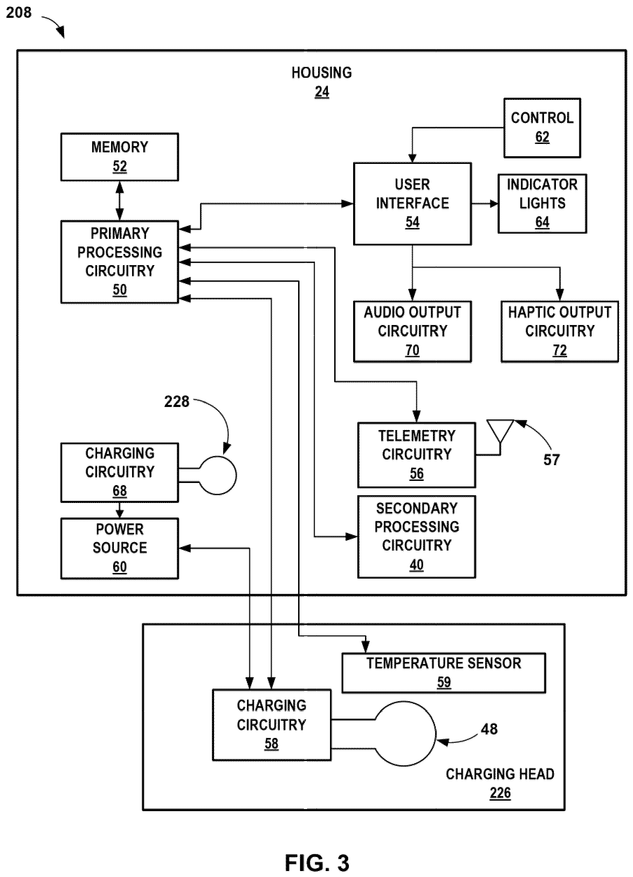

As noted above, external computing device 108 may also be referred to as charging device 108. As discussed further in connection with FIG. 3, charging device 108 may include a user interface to receive control inputs from a user, such as the patient, medical professional or other caregiver. The user interface of charging device 108 may also provide information to a user. For example, charging device 108 may include a control configured to receive user input (not shown in FIG. 1) as well as a set of indicator lights. In some examples the indicator lights may be configured to illuminate the control. The indicator lights may also be configured to output information regarding an operational state of external computing device 108, such as a communication status and wireless power transfer status.

As discussed further in connection with FIG. 3, charging device 108 includes processing circuitry configured to perform one or more processes related to charging device 108. In some examples, the processing circuitry determines whether IMD 10 and charging device 108 have established a communication link e.g., via communication circuitry. In response to the processing circuitry determining that charging device 108 and IMD 10 have not established a communication link, the processing circuitry may cause a notification to be generated. Charging device 108 may still wirelessly transfer power to IMD 10, but the notification may signify that charging device 108 is operating in open loop charging mode.

Processing circuitry of charging device 108 may further determine whether IMD 10 is receiving wireless power. In response to determining that IMD 10 has good power coupling, such as receiving an amount of wireless power above a power threshold, the processing circuitry may cause a notification to be generated.

Processing circuitry of system 100, e.g., processing circuitry of charging device 108, processing circuitry of server 112, and/or processing circuitry of IMD 10, may determine (e.g., calculate, receive, lookup, etc.) any of the values described herein.

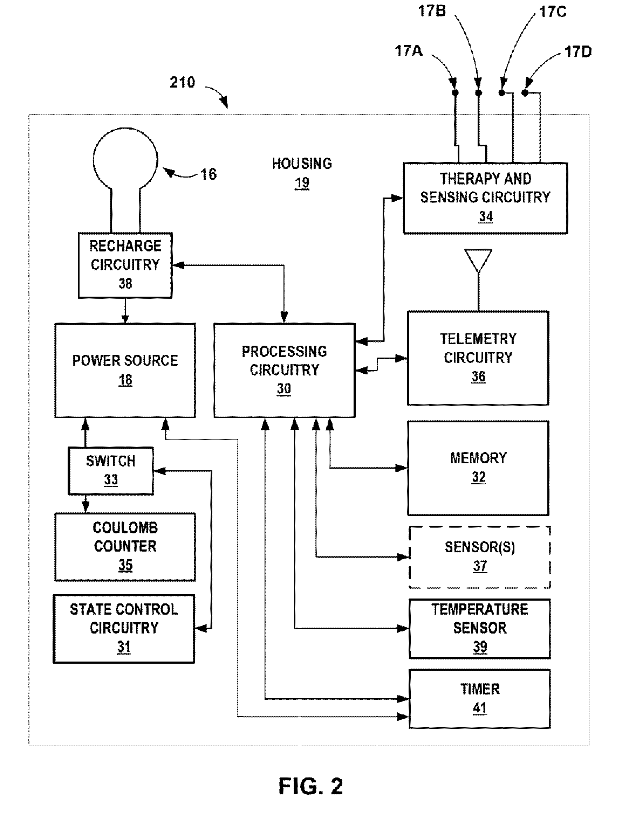

FIG. 2 is a block diagram illustrating example components of the medical device of FIG. 1. Implantable medical device (IMD) 210 is an example of IMD 10 described above in relation to FIG. 1. In the example illustrated in FIG. 2, IMD housing 19 of IMD 210 encloses temperature sensor 39, secondary coil 16, processing circuitry 30, therapy generation and sensing circuitry 34, recharge circuitry 38, memory 32, telemetry circuitry 36, power source 18, switch 33, coulomb counter 35, state control circuitry 31, timer 41, and, in some examples, one or more sensors 37, such as an accelerometer. In other examples, IMD 210 may include a greater or a fewer number of components, e.g., in some examples, IMD 210 may not include temperature sensor 39 or sensors 37. In general, IMD 210 may comprise any suitable arrangement of hardware, alone or in combination with software and/or firmware, to perform the various techniques described herein attributed to IMD 210 and processing circuitry 30, and any equivalents thereof.

Processing circuitry 30 of IMD 210 may include one or more processors, such as one or more microprocessors, digital signal processors (DSPs), application specific integrated circuits (ASICs), field programmable gate arrays (FPGAs), or any other equivalent integrated or discrete logic circuitry, as well as any combinations of such components. IMD 210 may include a memory 32, such as random-access memory (RAM), read only memory (ROM), programmable read only memory (PROM), erasable programmable read only memory (EPROM), electronically erasable programmable read only memory (EEPROM), flash memory, comprising executable instructions for causing the processing circuitry 30 to perform the actions attributed to this circuitry. Moreover, although processing circuitry 30, therapy generation and sensing circuitry 34, recharge circuitry 38, telemetry circuitry 36, temperature sensor 39, state control circuitry 31, coulomb counter 35, switch 33, and timer 41 are described as separate modules, in some examples, some combination of processing circuitry 30, therapy generation and sensing circuitry 34, recharge circuitry 38, telemetry circuitry 36, temperature sensor 39, state control circuitry 31, coulomb counter 35, switch 33, and timer 41 are functionally integrated. In some examples, processing circuitry 30, therapy generation and sensing circuitry 34, recharge circuitry 38, telemetry circuitry 36, and temperature sensor 39, state control circuitry 31, coulomb counter 35, switch 33, and timer 41 correspond to individual hardware units, such as ASICs, DSPs, FPGAs, or other hardware units. In this disclosure, therapy generation and sensing circuitry 34 may be referred to as therapy generation circuitry 34, for simplicity.

Memory 32 may store therapy programs or other instructions that specify therapy parameter values for the therapy provided by therapy generation circuitry 34 and IMD 210. In some examples, memory 32 stores one or more therapy schedules and/or data relating to the transition between therapy schedules. In some examples, memory 32 may also store temperature data from temperature sensor 39, instructions for recharging rechargeable power source 18, thresholds, instructions for communication between IMD 210 and an external computing device, or any other instructions required to perform tasks attributed to IMD 210. Memory 32 may be configured to store instructions for communication with and/or controlling one or more temperature sensors of temperature sensor 39. In various examples, memory 32 stores information related to determining the temperature of housing 19 and/or exterior surface(s) of housing 19 of IMD 210 based on temperatures sensed by one or more temperature sensors, such as temperature sensor 39, located within IMD 210.

In some examples, memory 32 stores programming settings such as electrical stimulation therapy output magnitude, pulse width, as well as other therapy parameters for one or more therapy programs and/or therapy schedules. Memory 32 may determine whether a sensed bioelectrical signal is valid, such as an evoked compound action potential (ECAP) or other signal in response to an output electrical stimulation therapy event. Memory 32 may store programming instructions that when executed by processing circuitry 30 cause processing circuitry 30 to cause therapy generation circuitry 34 to deliver electrical stimulation therapy to a target nerve of a patient.

In some examples, memory 32 stores data related to power source 18. In some examples, memory 32 stores data of one or more instances of therapy delivery, a status of power source 18 (e.g., an estimated level of charge remaining or measured level of charge remaining), predicted future use, and/or drain of power source 18.

Therapy generation and sensing circuitry 34 may generate and deliver electrical stimulation under the control of processing circuitry 30. In some examples, processing circuitry 30 controls therapy generation circuitry 34 by accessing memory 32 to selectively access and load at least one of the stimulation programs to therapy generation circuitry 34. For example, in operation, processing circuitry 30 may access memory 32 to load one of the stimulation programs to therapy generation circuitry 34. In such examples, relevant stimulation parameters may include a voltage amplitude, a current amplitude, a pulse rate, a pulse width, a duty cycle, or the combination of electrodes 17A, 17B, 17C, and 17D (collectively "electrodes 17") that therapy generation circuitry 34 may use to deliver the electrical stimulation signal as well as sense biological signals. In other examples, IMD 210 may have more or fewer electrodes than the four shown in the example of FIG. 2. In some examples, electrodes 17 may be part of or attached to a housing of IMD 210, e.g., a leadless electrode. In other examples, one or more of electrodes 17 may be part of a lead implanted in or attached to a patient to sense biological signals and/or deliver electrical stimulation, as described above in relation to FIG. 1.

In some examples, one or more electrodes 17 connected to therapy generation circuitry 34 may connect to one or more sensing electrodes, e.g., attached to housing of IMD 210. In some examples, electrodes 17 may be configured to detect the evoked motor response caused by the electrical stimulation therapy event, or other bioelectrical signals such as ECAPs, impedance, or other signals as appropriate.

IMD 210 also includes components to receive power to recharge rechargeable power source 18 when rechargeable power source 18 has been at least partially depleted. As shown in FIG. 2, IMD 210 includes secondary coil 16 and recharge circuitry 38 coupled to rechargeable power source 18. Recharge circuitry 38 may be configured to charge rechargeable power source 18 with the selected power level determined by either processing circuitry 30 or an external charging device, such as external computing device 108 described above in relation to FIG. 1. Recharge circuitry 38 may include any of a variety of charging and/or control circuitry configured to process or convert current induced in secondary coil 16 into charging current to charge power source 18.

Secondary coil 16 may include a coil of wire or other device capable of inductive coupling with a primary coil disposed external to a patient. Although secondary coil 16 is illustrated as a simple loop of in FIG. 2, secondary coil 16 may include multiple turns of conductive wire. Secondary coil 16 may include a winding of wire configured such that an electrical current can be induced within secondary coil 16 from a magnetic field. The induced electrical current may then be used to recharge rechargeable power source 18.

Recharge circuitry 38 may include one or more circuits that process, filter, convert and/or transform the electrical signal induced in the secondary coil to an electrical signal capable of recharging rechargeable power source 18. For example, in alternating current induction, recharge circuitry 38 may include a half-wave rectifier circuit and/or a full-wave rectifier circuit configured to convert alternating current from the induction to a direct current for rechargeable power source 18. The full-wave rectifier circuit may be more efficient at converting the induced energy for rechargeable power source 18. However, a half-wave rectifier circuit may be used to store energy in rechargeable power source 18 at a slower rate. In some examples, recharge circuitry 38 may include both a full-wave rectifier circuit and a half-wave rectifier circuit such that recharge circuitry 38 may switch between each circuit to control the charging rate of rechargeable power source 18 and temperature of IMD 210.

Rechargeable power source 18 may include one or more capacitors, batteries, and/or other energy storage devices. Rechargeable power source 18 may deliver operating power to the components of IMD 210. In some examples, rechargeable power source 18 may include a power generation circuit to produce the operating power. Rechargeable power source 18 may be configured to operate through many discharge and recharge cycles. Rechargeable power source 18 may also be configured to provide operational power to IMD 210 during the recharge process. In some examples, rechargeable power source 18 may be constructed with materials to reduce the amount of heat generated during charging. In other examples, IMD 210 may be constructed of materials and/or using structures that may help dissipate generated heat at rechargeable power source 18, recharge circuitry 38, and/or secondary coil 16 over a larger surface area of the housing of IMD 210. In some examples, power source 18 includes a non-rechargeable power source.

Although rechargeable power source 18, recharge circuitry 38, and secondary coil 16 are shown as contained within the housing of IMD 210, in other examples, at least one of these components may be disposed outside of the housing. For example, in some implementations, secondary coil 16 may be disposed outside of the housing of IMD 210 to facilitate better coupling between secondary coil 16 and the primary coil of external charging device. In other examples, power source 18 may be a primary power cell and IMD 210 may not include recharge circuitry 38 and secondary coil 16.

Processing circuitry 30 may also control the exchange of information with an external computing device using telemetry circuitry 36. Telemetry circuitry 36 may be configured for wireless communication using radio frequency (RF) protocols, such as Bluetooth, including Bluetooth low energy (BLE), or similar RF protocols, as well as using inductive communication protocols. Telemetry circuitry 36 may include one or more antennas configured to communicate with an external charging device (e.g., external computing device 108 of FIG. 1). Processing circuitry 30 may transmit operational information and receive therapy programs or therapy parameter adjustments via telemetry circuitry 36. Also, in some examples, IMD 210 may communicate with other implanted devices, such as stimulators, control devices, or sensors, via telemetry circuitry 36. In addition, telemetry circuitry 36 may be configured to control the exchange of information related to sensed and/or determined temperature data, for example temperatures sensed by and/or determined from temperatures sensed using temperature sensor 39. In some examples, telemetry circuitry 36 may communicate using inductive communication, and in other examples, telemetry circuitry 36 may communicate using RF frequencies separate from the frequencies used for inductive charging.

In some examples, processing circuitry 30 may transmit, via control of telemetry circuitry 36, additional information to external charging device related to the operation of rechargeable power source 18. For example, processing circuitry 30 may control telemetry circuitry 36 to transmit indications that rechargeable power source 18 is completely charged, rechargeable power source 18 is fully discharged, the amount of charging current output by recharge circuitry 38 e.g., to power source 18, or any other charge status of rechargeable power source 18. In some examples, processing circuitry 30 may use telemetry circuitry 36 to transmit instructions to external charging device, including instructions regarding further control of the charging session, for example instructions to lower the power level or to terminate the charging session, based on the determined temperature of IMD housing 19.

Processing circuitry 30 may also transmit information to external charging device that indicates any problems or errors with rechargeable power source 18 that may prevent rechargeable power source 18 from providing operational power to the components of IMD 210. In various examples, processing circuitry 30 may receive, through telemetry circuitry 36, instructions for algorithms, including formulas and/or values for constants to be used in the formulas, that may be used to determine the temperature of the housing 19 and/or exterior surface(s) of housing 19 of IMD 210 based on temperatures sensed by temperature sensor 39 located within IMD 210 during and after a recharging session performed on rechargeable power source 18.

IMD 210 also includes components for determining a status of power source 18. The status of power source 18 may be used for determination of a recharge interval of power source 18. For example, in examples where power source 18 includes a battery, IMD 210 may include components for determining (e.g., measuring, estimating, receiving, etc.) information related to a battery status, battery level, and/or other battery information (e.g., an amount of current drain from the battery, an amount of charge remaining in the battery, etc.). Components of IMD 210 for determining information related to the battery status include coulomb counter 35, switch 33, timer 41, and state control circuitry 31. Coulomb counter 35, switch 33, timer 41, and state control circuitry 31 may be used alone and/or in connection with other components of IMD 210, including processing circuitry 30.

In some examples, IMD 210 is configured to transition between different operational states. For example, processing circuitry 30 and/or state control circuitry 31 are configured to transition IMD 210 between different operational states where different components of IMD 210 are powered and operational. In a first device state (e.g., a therapy state or operational state) and/or during a first period of time, IMD 210 may be configured to deliver electrical stimulation therapy. Specifically, in the first device state, IMD 210 may be actually delivering therapy, or the components needed for IMD 210 to deliver therapy are receiving power but not actually delivering therapy (e.g., a device "standby" state). In a second device state, therapy is withheld or otherwise not scheduled, and IMD 210 may have one or more components disconnected or partially disconnected from power source 18 or otherwise in a state for consuming less power (e.g., a deep sleep state, a reduced power state, etc.). Processing circuitry 30 and/or state control circuitry 31 may also be configured to determine whether IMD 210 is in a given operational states. For example, processing circuitry 30 and/or state control circuitry 31 are configured to perform certain functions depending on whether IMD 210 is in a particular operational state (e.g., the first device state or the second devices state).

In some examples, IMD 210 uses one or more methods or combinations of components to determine the status of power source 18 of IMD 210 during different operational states. Using multiple methods of determining the status of power source 18 may optimize power loss from power source 18 while still maintaining a reliable determination of the status of power source 18.

In some examples, coulomb counter 35 is be configured to measure current drain from power source 18 (e.g., a battery) of IMD 210. Coulomb counter 35 may be configured to measure current drain during at least a first period of time in which the IMD is in a first device state (e.g., while the IMD is delivering electrical stimulation therapy via electrodes 17 or at least powered "on" and configured to delivery electrical stimulation therapy such as the "standby" state mentioned above). In some examples, coulomb counter 35 measures current drain directly from power source 18 (e.g., a battery) of IMD 210 during the first period of time. Coulomb counter 35 may output real-time current measurements that processing circuitry 30 uses to calculate a cumulative current used during a period of this may be part of or equal to the first period of time. In other examples, coulomb counter 35 may output an average current and/or cumulative current over a period of time to processing circuitry 30 for determining the current drain during the first period of time. Because coulomb counter 35 directly measures current drain from power source 18 (e.g., a battery), the measured current may be more accurate as compared to other methods of determining current drain (e.g., methods involving indirect measurement and/or estimation of current drain). Processing circuitry 30 may be configured to receive the measured current drain, or one or more values indicative of the measure current drain, from coulomb counter 35. In this way, processing circuitry 30 is configured to determine a recharge interval based on directly measuring battery current, alone or in combination with past or predicted current drain from power source 18.

As another example, IMD 210 may additionally or alternatively use more energy-efficient methods for estimating a battery status during a second period of time in which IMD 210 is in a second devices state (e.g., a deep sleep state). In some examples, high-power circuitry or components of IMD 210 may be disconnected from power source during the second device state, which may include one or more of a deep sleep state or a reduced power state. For example, state control circuitry 31 may be configured to disconnect coulomb counter 35 from power source 18 (e.g., the battery) via switch 33 (e.g., a circuitry switch). State control circuitry 31 may, in some examples, disconnect coulomb counter 35 from power source 18 by opening switch 33. In this way, IMD 210 may be able to conserve power during the second devices state because certain electrical components, including coulomb counter 35, are disconnected from power source 18. However, disconnecting coulomb counter 35 from power source 18 also disables coulomb counter 35 from directly measuring current drain during the second devices state (e.g., a deep sleep state). In some examples, coulomb counter 35 may be connected in order to measure current drain from power source 18 during at least a portion of the second devices state (e.g., a deep sleep state). Processing circuitry 30 may control state control circuity 31 to operate switch 33, or processing circuitry 30 may directly control switch 33 in other examples.

In some examples, one or more components of IMD 210 (e.g., processing circuitry 30, alone in combination with other components) may be configured to estimate current drain from power source 18 (e.g., battery) of IMD 210 for a second period of time during the second device state. As discussed above, this second period of time may include the coulomb counter 35 being disconnected and not available to measure the current drain from power source 18. For example, where a coulomb counter 35 does not measure current drain from power source 18, one or more techniques may be used to estimate the current drain from power source 18. In some examples, processing circuitry 30, alone or in combination with other components, are configured to determine (e.g., calculate) an estimated current drain based on at least information indicative of a characterized current drain and information indicative of IMD 210 events. The information indicative of the characterized current drain, as well as information indicative of IMD 210 events, may enable IMD 210 to estimate (e.g., via a calculation) current drain from power source 18 (e.g., battery) during the second devices state (e.g., a deep sleep state). For example, the information indicative of the characterized current drain includes an amount of current drain per unit of time (e.g., amperes per hour), such that processing circuitry 30 determines current drain during the second devices state (e.g., a deep sleep state) based on the amount of time spent in the second device state and this expected, or estimated, current drain or other indication of battery usage. In some examples, processing circuitry 30 accesses information indicative of the characterized current drain from memory 32, programmer 104, server 112, or another suitable component. Processing circuitry 30 may be configured to receive information indicative of IMD 210 events from timer 41. To determine the estimated current drain during the second device state, processing circuitry 30 may be configured to access a lookup table, wherein the lookup table correlates at least the information indicative of IMD 210 events and the information indicative of the characterized current drain from power source 18.

In some examples, timer 41 is configured to provide information indicative of IMD 210 events, which may facilitate and/or enable determination of an estimated current drain during the second devices state (e.g., a deep sleep state). Timer 41 may remain powered and operational during at least the second devices state (e.g., a deep sleep state), but may also remain operation during the first device state (e.g., when IMD 210 is configured to deliver therapy). In some examples, timer 41 records the duration of time IMD 210 is in the second devices state (e.g., a deep sleep state). Additionally, or alternatively, timer 41 records when IMD 210 transitions between the first device state (e.g., when IMD 210 is configured to deliver therapy) and the second devices state (e.g., a deep sleep state) and vice versa. For example, timer 41 records one or more timestamps of at least one of IMD 210 entering or exiting the second device state. In this way, timer 41 may provide a processor, such as processing circuitry 30, with timestamps of when IMD 210 transitions between the first device state (e.g., when IMD 210 is configured to deliver therapy) and the second devices state (e.g., a deep sleep state), which may enable processing circuitry 30 to determine (e.g., calculate) the duration of time spent in the first device state or the second device.

Although timer 41 provides information indicative of IMD 210 events in the example of FIG. 2, another suitable component may provide information indicative of IMD 210 events. For example, an external device (e.g., external computing device 108, programmer 104, and/or server 112 of FIG. 1) to IMD 210 may record information indicative of IMD 210 events. For example, external computing device 108, programmer 104, and/or server 112 records the duration of time IMD 210 is in the second devices state (e.g., a deep sleep state). The external device (e.g., external computing device 108, programmer 104, and/or server 112 of FIG. 1) may be configured to send the information indicative of IMD 210 events to IMD 210.

In addition, or alternative, to the methods of determining a current drain from power source 18 and/or a status of power source 18 noted above, IMD 210, via processing circuitry 30, may be configured to measure a voltage of power source 18 (e.g., a battery) as part of determining the battery status or battery usages.

IMD 210, via processing circuitry 30, may be configured to determine a status of power source 18 (e.g., a battery status, in examples where power source 18 includes at least a battery). The determination of the status of power source 18 (e.g., a battery status) may be based on the measured current drain and the estimated current drain of the battery of the IMD for different respective periods of time in some examples. However, the status of power source 18 may be determined using only estimated current drain or only measured current drain in other examples. IMD 210, via processing circuitry 30, may be configured to generate, for output, information indicative of the status of power source 18 (e.g., a battery status, in examples where power source 18 includes at least a battery). Information indicative of the status of power source 18 (e.g., a battery status) may include an indication of at least one of an amount (e.g., a percentage) of remaining charge, a time until recharge, a recharge interval, a date of charge depletion, an expected date of battery depletion, an expected date of battery recharge. In some examples, IMD 210, via processing circuitry 30, may be configured generate, for output, information indicative of the status of power source 18 at predetermined events (e.g., battery percentage thresholds of remaining charge, such as 20 percent, 10 percent, etc.). The information indicative of the status of power source 18 may be updated automatically on a periodic basis, after one or more events (e.g., a recharge session, the start or end of a therapy session, etc.), or upon interrogation of IMD 210 by an external device (e.g., programmer 104, external computing device 108, or server 112).

FIG. 3 is a block diagram of an example an external computing device of FIG. 1. External charging device 208 in of FIG. 3 is an example of external computing device 108 described above in relation to FIG. 1. In some examples, external charging device 208 may be described as a hand-held device, in other examples, external charging device 208 may be a larger or a non-portable device. In addition, in other examples external charging device 208 may be included as part of an external programmer or include functionality of an external programmer. As shown in the example of FIG. 3, external charging device 208 includes a housing 24 connected to a charging head 226. Housing 24 encloses components such as a primary processing circuitry 50, memory 52, user interface 54, telemetry circuitry 56, control 62, one or more sets of indicator lights 64, audio output circuitry 70, haptic output circuitry 72 and power source 60. Charging head 226 may include charging circuitry 58, temperature sensor 59, and external primary coil 48. Charging head 226 and/or external primary coil 48 may be an example of external primary coil 26 as shown in FIG. 1. Housing 24 is electrically coupled to charging head 226 via a cable. Housing 24 may also include charging circuitry 68 and internal primary coil 228, which is an example of internal primary coil 28 described above in relation to FIG. 1.

In some examples, separate charging head 226 may facilitate positioning of external primary coil 48 over secondary coil 16 of IMD 10 (as shown in FIG. 1) or IMD 210 (as shown in FIG. 2). In some examples, charging circuitry 68 and/or internal primary coil 228 may be integrated within housing 24. In other examples, external charging device 208 may not include charging head 226. Memory 52 may store instructions that, when executed by primary processing circuitry 50, causes primary processing circuitry 50 and external charging device 208 to provide the functionality ascribed to external charging device 208 throughout this disclosure, and/or any equivalents thereof. External primary coil 48 and internal primary coil 228 may also be referred to as an antenna. In some examples, external charging device 208 may include secondary processing circuitry 40, which may control telemetry circuitry 56, as well as perform other functions. Some other functions may include error checking of the operation of primary processing circuitry 50.

External charging device 208 may also include one or more temperature sensors, illustrated as temperature sensor 59 within charging head 226, similar to temperature sensor 39 of FIG. 2. As shown in FIG. 3, temperature sensor 59 may be disposed within charging head 226. In other examples, one or more temperature sensors of temperature sensor 59 may be disposed within housing 24. For example, charging head 226 may include one or more temperature sensors positioned and configured to sense the temperature of external primary coil 48 and/or a surface of the housing of charging head 226. In some examples, external charging device 208 may not include temperature sensor 59.

In general, external charging device 208 comprises any suitable arrangement of hardware, alone or in combination with software and/or firmware, to perform the techniques ascribed to external charging device 208, and primary processing circuitry 50, user interface 54, telemetry circuitry 56, and charging circuitry 68 of external charging device 208, and/or any equivalents thereof. In various examples, external charging device 208 may include one or more processors, such as one or more microprocessors, DSPs, ASICs, FPGAs, or any other equivalent integrated or discrete logic circuitry, as well as any combinations of such components. External charging device 208 also, in various examples, may include a memory 52, such as RAM, ROM, PROM, EPROM, EEPROM, flash memory, a hard disk, a CD-ROM, comprising executable instructions for causing the one or more processors to perform the actions attributed to them. Moreover, although primary processing circuitry 50, telemetry circuitry 56, charging circuitry 68, and temperature sensor 59 are described as separate modules, in some examples, primary processing circuitry 50, telemetry circuitry 56, charging circuitry 68, and/or temperature sensor 59 are functionally integrated. In some examples, primary processing circuitry 50, telemetry circuitry 56, charging circuitry 68, and/or temperature sensor 59 correspond to individual hardware units, such as ASICs, DSPs, FPGAs, or other hardware units.

Memory 52 may store instructions that, when executed by primary processing circuitry 50, cause primary processing circuitry 50 and external charging device 208 to provide the functionality ascribed to external charging device 208 throughout this disclosure, and/or any equivalents thereof. For example, memory 52 may include instructions that cause primary processing circuitry 50 to control the power level used to charge IMD 210 in response to the determined temperatures for the housing/external surface(s) of IMD 210, as communicated from IMD 210, or instructions for any other functionality. Memory 52 may include a record of selected power levels, sensed temperatures, determined temperatures, or any other data related to charging rechargeable power source 18, described above in relation to FIG. 2. Memory 52 may store instructions that when executed by primary processing circuitry 50 may control the operation of indicator lights 64 as described above in relation to FIG. 1. Primary processing circuitry 50 may determine one or more operational states, e.g., of external charging device 208 and selectively control indicator lights 64 based on the operational state.

Primary processing circuitry 50 may, when requested, transmit any stored data in memory 52 to another computing device for review or further processing, such as to server 112 depicted in FIG. 1. Primary processing circuitry 50 may be configured to access memory, such as memory 32 of IMD 10 and/or memory 52 of external charging device 208, to retrieve information comprising instructions, formulas, and determined values for one or more constants.

User interface 54 may include buttons, such as control 62 or a keypad, lights, such as indicator lights 64, a speaker for voice commands, a display, such as a liquid crystal display (LCD), light-emitting diode (LED), or cathode ray tube (CRT). In some examples, the display may be a touch screen. Control 62 may be implemented as any type of component that may receive user input and provide an indication of the user input to primary processing circuitry 50. Control 62 may be a knob, switch, button or another suitable structure. As discussed in this disclosure, primary processing circuitry 50 may present and receive information relating to the charging and/or the status of rechargeable power source 18 (e.g., a battery) of IMD 210 via user interface 54. For example, user interface 54 may indicate when charging is occurring, quality of the alignment between internal primary coil 228 or external primary coil 48 and secondary coil 16 of IMD 210, the selected power level, current charge level of rechargeable power source 18, duration of the current recharge session, anticipated remaining time of the charging session, sensed temperatures, or any other information. Primary processing circuitry 50 may receive some of the information displayed on user interface 54 from IMD 210 in some examples. In some examples, user interface 54 may provide an indication to the user of the status of power source 18 of IMD 210. For example, user interface 54 may provide information indicative of the status of power source 18 (e.g., a battery status) including an indication of at least one of an amount (e.g., a percentage) of remaining charge, a time until recharge, a recharge interval, an expected date of battery depletion, or an expected date of battery recharge.

User interface 54 may also receive user input via user interface 54. The input may be, for example, in the form of pressing a button on a keypad or selecting an icon from a touch screen. The input may change programmed settings, start or stop therapy, request starting or stopping a recharge session, a desired level of charging, or one or more statistics related to charging rechargeable power source 18 (e.g., the cumulative thermal dose). In this manner, user interface 54 may allow the user to view information related to the operation of IMD 210. For example, control 62 may provide an input to primary processing circuitry 50 to cause primary processing circuitry 50 to start or stop delivery of wireless power to the power receiving device, e.g., IMD 10 or IMD 210 described above in relation to FIGS. 1 and 2.

Charging circuitry 58 may include one or more circuits that generate an electrical signal, and an electrical current, within external primary coil 48. Charging circuitry 58 may generate an alternating current of specified amplitude and frequency in some examples. In other examples, charging circuitry 58 may generate a direct current. In any case, charging circuitry 58 may be capable of generating electrical signals, and subsequent magnetic fields, to transmit various levels of power to IMD 210. In this manner, charging circuitry 58 may be configured to charge rechargeable power source 18 of IMD 210 with the selected power level.

Power source 60 may deliver operating power to the components of external charging device 208. Power source 60 may also deliver the operating power to drive external primary coil 48 during the charging process. Power source 60 may include a battery and a power generation circuit to produce the operating power. In some examples, a battery of power source 60 may be rechargeable to allow extended portable operation. In other examples, power source 60 may draw power from a wired voltage source such as a consumer or commercial power outlet.

Telemetry circuitry 56 supports wireless communication between IMD 210 and external charging device 208 under the control of primary processing circuitry 50. Telemetry circuitry 56 may also be configured to communicate with another computing device via wireless communication techniques, or direct communication through a wired connection. In some examples, telemetry circuitry 56 may be substantially similar to telemetry circuitry 36 of IMD 210 described herein, providing wireless communication via an RF or proximal inductive medium. In some examples, telemetry circuitry 56 includes an antenna 57, which may take on a variety of forms, such as an internal or external antenna. Although telemetry circuitry 56 and telemetry circuitry 36 may each include dedicated antennas for communications between these devices, telemetry circuitry 56 and telemetry circuitry 36 may instead, or additionally, be configured to utilize inductive coupling from internal primary coil 228 and/or external primary coil 48 to transfer data.

Examples of local wireless communication techniques that may be employed to facilitate communication between external charging device 208 and IMD 210 include radio frequency and/or inductive communication according to any of a variety of standard or proprietary telemetry protocols, or according to other telemetry protocols such as the Institute of Electrical and Electronics Engineers (IEEE) 802.11x or Bluetooth specification sets. In this manner, other external devices may be capable of communicating with external charging device 208 without needing to establish a secure wireless connection.

In operation, primary processing circuitry 50, and/or secondary processing circuitry 40, may control one or more sets of indicator lights 64 to provide information to a user about communication, charging efficiency, therapy status of the IMD, or other applicable information. For example, primary processing circuitry 50 may determine whether communication circuitry, e.g., telemetry circuitry 56, has established a communication link with a power receiving device (e.g., IMD 10 or IMD 210 depicted in FIGS. 1 and 2). Primary processing circuitry 50 may also determine whether the power receiving device (e.g., IMD 10 or IMD 210) is receiving wireless power, e.g., via charging circuitry 68 and internal primary coil 228, or charging circuitry 58 and external primary coil 48.

Primary processing circuitry 50 may use any one or more system metrics to determine power transfer to IMD 210. In some examples, IMD 210 may send a signal indicating an amount of current output by the recharge circuitry of IMD 210. In other examples, primary processing circuitry 50 may calculate other system metrics, such as alignment of internal primary coil 228 to secondary coil 16 of IMD 210 using any of several techniques, including heat calculations, temperature measurements, detection of metal, and/or other suitable determinations. Primary processing circuitry 50 may compare any of the calculated power transfer, power efficiency, alignment, IMD 210 current, etc. to a threshold stored at memory 52. When above the threshold, primary processing circuitry 50 may cause indicator lights 64 to output a signal.

In some examples, primary processing circuitry 50 of external charging device 208 may be configured to determine the operational states of IMD 210. In some examples, primary processing circuitry 50 of external charging device 208 is configured to determine whether IMD 210 is in the first device state (e.g., when IMD 210 is configured to deliver therapy) and/or the second devices state (e.g., a deep sleep state). Primary processing circuitry 50 of external charging device 208 may determine the operational state of IMD 210 instead of or in addition to processing circuitry 30 of IMD 210 as described above. Further, primary processing circuitry 50 of external charging device 208 may be configured to perform any of the functions related to determining a status of power source 18 of IMD 210, as well as additionally or alternatively determining a status of power source 60 of external charging device 208.

In some examples, primary processing circuitry 50 may control haptic output circuitry 72 to provide a tactile sensation above the patient's perception level. For example, haptic output circuitry may vibrate or provide some similar tactile sensation. In some examples, primary processing circuitry 50 may control haptic output circuitry 72 to vibrate at a constant level for a specified duration, may output a pattern of vibration, or some similar haptic feedback for the patient. In some examples, the haptic feedback may indicate poor coupling, and the haptic feedback may fade as the coupling improves, e.g., the power receiving device is receiving wireless power above the first threshold. In this manner, the patient may receive feedback without the need to view user interface 54 of external charging device 208, or the user interface of some other device, e.g., a smart phone, tablet and so on. As discussed above, primary processing circuitry 50 may be configured to provide similar notifications or outputs related to the determination of the status of power source 18 of IMD 210. For example, primary processing circuitry 50 may control haptic output circuitry 72 to vibrate in a specific pattern to indicate to and/or alert the patient of the status of power source 18 of IMD 210.

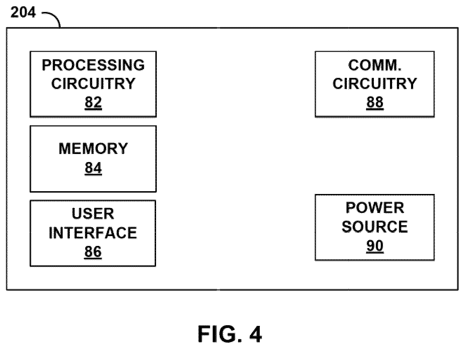

FIG. 4 is a block diagram of an example programmer of FIG. 1. Programmer 204 may be a device for inputting information relating to a patient, receiving information from IMD 210, and updating IMD 210. In some examples, such as where programmer 204 is a patient programmer, programmer 204 can be a wearable communication device, with a therapy request input integrated into a key fob or a wristwatch, handheld computing device, smart phone, computer workstation, or networked computing device. Programmer 204 can be a bring-your-own device provided by the patient, or provided by the healthcare provider in connection with the implantable device.

In some examples, such as where programmer 204 is physician/clinician programmer, programmer 204 is a tablet computing device that is preloaded with a specific application to interface with IMD 210. The physician or clinician may interact with programmer 204 for programming IMD 210. The physician or clinician may utilize programmer 204 to program IMD 210 (e.g., program therapy parameters, such as for one or more therapy schedules), as well as view information about the usage of IMD 210.

Programmer 204 generally comprises a processing circuitry 82, a memory 84, a user interface 86, communications circuitry 88, and a power source 90. Processing circuitry 82 can be any programmable device that accepts digital data as input, is configured to process the input according to instructions or algorithms, and provides results as outputs. In an example, processing circuitry 82 can be a central processing unit (CPU) configured to carry out the instructions of a computer program. Processing circuitry 82 is therefore configured to perform at least basic arithmetical, logical, and input/output operations. In one or more examples, processing circuitry 82 corresponds to individual hardware units, such as microprocessors, ASICs, DSPs, FPGAs, or other hardware units. In other examples, processing circuitry 82 can correspond to multiple individual hardware units, such as microprocessors, ASICs, DSPs, FPGAs, or other hardware units.

Memory 84 can comprise volatile or non-volatile memory as required by processing circuitry 82 to not only provide space to execute the instructions or algorithms, but to provide the space to store the instructions themselves. In one or more examples, volatile memory can include RAM, DRAM, or static random access memory (SRAM), for example. In one or more examples, non-volatile memory can include read-only memory, flash memory, ferroelectric RAM, hard disk, floppy disk, magnetic tape, or optical disc storage, for example. The foregoing lists in no way limit the type of memory that can be used.

User interface 86 can include a button or keypad, lights, a speaker for voice commands, a knob able to turn, a display, such as a liquid crystal display (LCD), light-emitting diode (LED), or cathode ray tube (CRT). In some examples, the display may be a touch screen. Processing circuitry 82 can present and receive information relating to electrical stimulation and resulting therapeutic effects via user interface 86. For example, processing circuitry 82 can receive patient input via user interface 86. The input can be, for example, in the form of pressing a button on a keypad or selecting an icon from a touch screen. Processing circuitry 82 can also present information to the patient in the form of alerts related to delivery of the electrical stimulation to a patient or a caregiver via user interface 86. In some examples, user interface 86 is configured to provide a status of power source 18 of IMD 210. For example, user interface 86 may provide information indicative of the status of power source 18 (e.g., a battery status) including an indication of at least one of an amount (e.g., a percentage) of remaining charge, a time until recharge, a recharge interval, an expected date (e.g., including month, day, and year) of battery depletion, an expected date (e.g., including month, day, and year) of battery recharge, and/or a suggested recharge interval according to the techniques of this disclosure.

Communications circuity 88 is configured to interface with IMD 210 and optionally, server 112 (FIG. 1). Communications circuity 88 supports wireless communication between IMD 210 and, optionally, between server 112 and programmer 204 under the control of processing circuitry 82. Communications circuity 88 can also be configured to communicate with another computing device via wireless communication techniques, or direct communication through a wired connection. Communications circuity 88 can provide wireless communication via an RF or proximal inductive medium. In some examples, communications circuity 88 can include an antenna, which may take on a variety of forms, such as an internal or external antenna.

Examples of local wireless communication techniques that may be employed to facilitate communication between programmer 204 and another computing device include RF communication according to the 802.11 or Bluetooth specification sets, infrared communication, e.g., according to the Infrared Data Association (IrDA) standard, or other standard or proprietary telemetry protocols. In this manner, other external devices may be capable of communicating with programmer 204 without needing to establish a secure wireless connection.

Power source 90 delivers operating power to the components of programmer 204. Power source 90 can include a battery and a power generation circuit to produce the operating power. In some examples, the battery may be rechargeable by for example, an exterior power source.

Programmer 204 allows the user (e.g., patient, caretaker, clinician, physician) to program one or more therapy schedules and therapy parameters (e.g., amplitude, frequency, pulse width, and/or the like) according to one or more therapy programs. A therapy schedule may include a frequency and duration of stimulation therapy (e.g., electrical stimulation therapy) based on certain time intervals (e.g., times of the day, number of days between therapy sessions, particular dates and/or days of the week for therapy sessions, total duration and/or number of therapy sessions, etc.). Programmer 204 can communicate with IMD 210 to update the functionality of IMD 210. In this way, programmer, e.g., via processing circuitry 82, is configured to control IMD 210 to deliver electrical stimulation therapy, including therapy to one or more of a sacral nerve or tibial nerve for incontinence therapy according to the one or more therapy programs. In some examples, programmer 204 is configured to communicate with external computing device 208, which may in turn communicate with IMD 210 for programming and/or controlling IMD 210.

In some examples, programmer 204 is configured to perform one or more of the functions related to determining a status of power source 18 (e.g., a battery status) of IMD 210, as described above. For example, programmer 204, via processing circuitry 82, may be configured to estimate of the status of power source 18 for one or more periods of time and/or one or more device states of IMD 210. In some examples, programmer 204 is configured to track and/or estimate the status of power source 18 (e.g., a battery status) even when not connected (e.g., physically and/or communicatively connected) to IMD 210. Programmer 204 may provide notifications related to an expected status of power source 18 of IMD 210. IMD 210 may provide updated information related to the status of power source 18 to programmer 204.

In accordance with the techniques of this disclosure, programmer 204, via processing circuitry 82, is configured to determine a recharge interval (e.g., a recharge interval value) for power source 18 of IMD 210 and determine, based on the recharge interval, a suggested recharge interval (e.g., a suggested recharge interval value) for power source 18 of IMD 210. For example, programmer 204, via communication circuitry 88, receives information indicative of the status of power source 18 of IMD 210 to determine the recharge interval. The recharge interval may be based on a status (e.g., an instantaneous status or current status) of power source 18, a historical use of power source 18, and/or predicted future use according to one or more therapy schedules.

As discussed in connection with previous examples, the information indicative of the status of power source 18 can include indications of at least one of an amount (e.g., a percentage) of remaining charge, a time until recharge, a date of charge depletion, an expected date of battery depletion, an expected date of battery recharge, or the actual recharge interval value itself.

Programmer 204, via processing circuitry 82, may perform one or more calculations, manipulations, or other operations with information indicative of the status of power source 18 to determine the recharge interval, which may include multiple recharge intervals for multiple different therapy schedules. Ultimately, programmer 204, via processing circuitry 82, determines the suggested recharge interval, which may be the same or different than the determined (e.g., calculated) recharge interval value. The suggested recharge interval, which programmer 204 may generate for presentation to a user, such as via user interface 86, may be a value that is easy for a user to remember and may align with time intervals that would normally occur on a patient's schedule or are otherwise easier for a user to remember.

In general, programmer 204, via communication circuitry 88, may be configured to receive information from IMD 210 and/or external computing device 208 for determining the calculated recharge interval and/or the suggested recharge interval. In some examples, processing circuitry 82 receives information of at least one therapy program for IMD 210. The information of each therapy program can include, but is not limited to, information about therapy parameters and/or information about one or more therapy schedule defining delivery of therapy by IMD 210. As discussed in connection with previous examples, therapy parameters may include one or more of an amplitude, frequency, and/or pulse width, as well as other suitable parameters. Therapy parameters such as amplitude, frequency, and/or pulse width, as well as other suitable parameters may cause the recharge interval to be longer or shorter depending on the amount of energy used from the power source 18 of IMD 210. For example, a recharge interval may vary between a few days (e.g., one day, two days, three days, etc.) to many months (e.g., 12 month, 18 months, 24 months, 36 months, or more) depending how therapy program parameters are configured. A therapy schedule may include a frequency and duration of stimulation therapy using one or more sets of stimulation parameters and based on certain time intervals (e.g., times of the day, number of days between therapy sessions, particular dates and/or days of the week for therapy sessions, total duration and/or number of therapy sessions, etc.).

In some examples, processing circuitry 82 is configured to determine information of each therapy program based on historical data for a given patient. For example, processing circuitry 82 determines therapy program information based on one or more instances of therapy actually delivered to the patient (e.g., wherein therapy actually delivered to the patient may the same or different as compared to the therapy program). In some examples, processing circuitry 82 receives information of from one or more sensors of IMD 210 (e.g., one or more of sensors 37) over one or more instances of therapy actually delivered to the patient to determine therapy program information (e.g., as part of a closed-loop therapy system). For examples, processing circuitry 82 determines therapy parameters based on physiological sensing via one or more sensors 37.

In some examples, the received information from IMD 210 (e.g., information of a therapy program) additionally or alternatively includes therapy dose information. Therapy dose information can include information about historical and/or predicted future delivery of therapy by IMD 210 to the patient. Therapy dose information may define delivery of therapy relative to time, such as for a given instance of therapy delivery within a therapy schedule. For example, therapy dose information may indicate one or more of continuous stimulation, cycled stimulation, or other configurations of therapy related to the amount of therapy (e.g., stimulation) over time. Processing circuitry 82 may use dose information instead of, or in combination with, information of a therapy schedule to determine recharging information (e.g., a recharge interval value and/or suggested recharge interval). For example, some systems (e.g., some DBS, SCS systems) may be configured to deliver therapy according to therapy dose information (e.g., continuous therapy, cycled stimulation, etc.) rather than according a predefined therapy schedule. Such systems (e.g., DBS, SCS systems, etc.) may be configured to use therapy dose information for determining recharging information (e.g., a recharge interval value and/or suggested recharge interval). In some examples, processing circuitry 82 is configured to determine therapy dose information based on historical data for a given patient. For example, processing circuitry 82 determines therapy dose information based on one or more instances of therapy actually delivered to the patient. In some examples, processing circuitry 82 receives information of from one or more sensors of IMD 210 (e.g., one or more of sensors 37) over one or more instances of therapy actually delivered to the patient to determine dose information.

In some examples, processing circuitry 82 receives information of multiple therapy programs, such as a first therapy program (e.g., which may include a first set of therapy parameters and/or a first therapy schedule, such as an induction schedule) and a second therapy program (e.g., which may include a second set of therapy parameters and/or a second therapy schedule, such as a maintenance schedule). However, processing circuitry 82 can be configured to receive information for any number of therapy programs, sets of therapy parameters, and/or therapy schedules. In some examples, as discussed relation other examples, processing circuitry receives information of a status of power source 18 of IMD 210 (which include one or more of a current status, a past status from a previous time, and/or a projected future status). A set of stimulation parameters generally defines a stimulation signal (e.g., continuous signal or set of pulses) that is delivered to the patient. A therapy program typically is defined by the set of stimulation parameters, and different therapy programs may differ by the value of at least one stimulation parameter of the set of stimulation parameters (e.g., at least one of an amplitude, pulse width, frequency, duty cycle, etc. may be different between different programs).

In some examples, each therapy program of the multiple therapy programs has unique therapy parameters and/or a unique schedule. For example, a first therapy program may include a first set of therapy parameters and a first schedule (e.g., which may include an induction schedule schedule) and a second therapy program may include a second set of therapy parameters and the second schedule (e.g., which may include a maintenance schedule). In some examples, the first set of therapy parameters differs from the second set of therapy parameters. In some the first set of therapy parameters is the same as the second set of therapy parameters (e.g., the first set of therapy parameters and the second set of therapy parameters are the same, but are delivered according to different therapy schedules, such as the first therapy schedule and the second therapy schedule).

In some examples, once programmer 204 has received information indicative of the therapy parameters and at least one therapy schedule (and/or therapy dose information), programmer 204, e.g., via processing circuitry 82, is configured to determine a recharge interval value (otherwise referred to as a "determined" or "calculated" recharge interval for purposes of this disclosure) of a power source of the implantable medical device (e.g., power source 18 of IMD 210) based on the therapy parameters and the therapy schedule. In some examples, processing circuitry 82 determines a recharge interval value for each therapy program of multiple therapy programs (e.g., such as in instances including at least an induction schedule, a maintenance schedule, and/or more additional schedules). The determined recharge interval value may include a time until power source 18 of IMD 210 needs to be recharged for a given therapy schedule. In some examples, the determined recharge interval value indicates a period of time for power source 18 of IMD 210 to deplete to and/or below a threshold level. In some examples, the determined recharge interval value indicates a period of time for power source 18 of IMD 210 to run out of charge, and/or not have enough energy to perform operational functions of IMD 210. For example, the recharge interval value may be expressed as a number of seconds, minutes, hours, days, weeks, months, years, or another suitable period of time until power source 18 of IMD 210 needs to be recharged. The determined recharge interval for a first therapy schedule (e.g., an induction schedule) may be different from a second therapy schedule (e.g., a maintenance schedule) because of the instances of therapy delivery are more or less frequent, include a greater amount of therapy delivered, and/or otherwise require different amounts of energy from power source 18 of IMD 210 (e.g., because of more or less sensing, feedback, interrogation, and/or other functions of IMD 210 performed during a first therapy schedule as opposed to the second therapy schedule).

In some examples, once programmer 204, e.g., via processing circuitry 82, has determined the calculated recharge interval for each therapy schedule of the one or more therapy schedule, programmer 204, e.g., via processing circuitry 82, determines a suggested recharge interval value from a plurality of predefined possible suggested recharge interval values. The predefined possible suggested recharge interval values may be pre-programmed and stored in memory 84, such as for access by processing circuitry 82. In some examples, processing circuitry 82 determines (e.g., calculates) a suggested recharge interval value from one of a plurality of predefined suggested recharge interval values. In some examples, processing circuitry 82 determines a suggested recharge interval value from a plurality of predefined possible suggested recharge interval values corresponding to a respective range of possible values of the determined recharge interval value. In some examples, the predefined suggested recharge interval values correspond to bins defined in terms of possible values of the determined recharge interval value. In some examples, each bin of the plurality of bins correlates a range of the possible values of the determined recharge interval value to one of the plurality of predefined possible suggested recharge interval values. In general, processing circuitry 82 determines a suggested recharge interval by determining the bin in which the calculated recharge interval resides, and selects the suggested recharge interval corresponding to that particular bin. Every bin of the multiple bins is defined in terms of possible values of the recharge interval value, and corresponds to a particular suggested recharge interval value. In this way, processing circuitry 82 determines the suggested recharge interval value based on the calculated recharge interval value and a plurality of bins. Said another way, processing circuitry 82 provides the calculated recharge interval value to a model or algorithm defining the bins, and the output of the model or algorithm is the suggested recharge interval for each therapy schedule of the one or more therapy schedules. In effect, processing circuitry 82 rounds a calculated recharge interval value to the predetermined suggested recharge interval value associated with a particular bin.

In some examples, each bin defines at least one of an upper bound value and a lower bound value, and some bins define both an upper bound value and a lower bound value. In some examples, the upper bound value is a maximum recharge interval value. In some examples the lower bound value minimum recharge interval value. In some examples, each bin of the multiple bins defines a range between the lower bound value and the upper bound value, e.g., such that a determined recharge interval value in the range corresponds to the suggested recharge interval associated with the particular bin of the multiple bins.

To determine the suggested recharge interval, processing circuitry 82 may perform one or more operations with the determined recharge interval value as it relates to the ranges of possible values of the determined recharge interval values and/or the bins. In some examples, processing circuitry 82 compares the determined recharge interval value to the range of the possible values of the determined recharge interval value for each bin of the plurality of bins. In some examples, based on the comparison, processing circuitry 82 selects the suggested recharge interval value that corresponds to the bin with the range of determined recharge interval values in which the determined recharge interval resides. In this way, processing circuitry 82 may be configured to select a particular bin corresponding to the determined recharge interval value, e.g., by determining the bin in which the determined recharge interval value resides.

In some examples, the suggested recharge interval value correlating to each range of the ranges of possible values of the determined recharge interval value and/or bin is lower than the lower bound value for the at least some of bins of the plurality of bins. For example, the suggested recharge interval value may include a buffer (e.g., a safety buffer) from the lower bound value of each bin, such that the suggested recharge interval value corresponding to a particular bin is lower than the lower bound determined recharging interval value for the particular bin. In some examples, the buffer may be a percentage of the lower bound value. In some examples, the suggested recharge interval value for a particular bin is at least twenty percent lower than the lower bound determined recharge value for the particular bin. In some examples, the buffer is the same for all bins. In some examples, the buffer is different for each bin. For example, the buffer for bins associated with lower suggested recharge interval (e.g., less than 1 month) may be greater than the buffer for bins associated with higher suggested recharge interval (e.g., greater than one month). In some examples, when the buffer value is calculated as a percentage of the lower bound value, the buffer value is capped at a particular time (e.g., such as a 2 month buffer cap, which may be applicable for bins with larger recharge intervals). In some examples, the buffer value is proportional to the suggested recharge interval for a given bin, such that bins associated with higher suggested recharge interval values include a larger buffer between the bin lower bound value and the suggested recharge interval value. In some examples, the buffer is a static value across all bins (e.g., a set number of minutes, hours, days, etc.), rather than being a percentage below the lower bound of any given bin.

Processing circuitry 82 may be configured to determine recharge interval values and/or suggested recharge interval values in different way in addition to or instead of techniques described above. In some examples, processing circuitry 82 determines suggested recharge interval values using historical data (e.g., records of instances of actual therapy delivery) and/or predicted future instances of therapy delivery. In some examples, processing circuitry 82 may determine suggested recharge interval values based on a model (e.g., empirically trained model), wherein inputs to the model include recharge interval values and outputs include one or more suggested recharge interval values. Additional inputs may include therapy programming parameters (e.g., therapy schedules and/or therapy parameters such as amplitude, frequency, and/or pulse width), patient-specific factors (e.g., age, gender, disease state, recharging interval preference), and/or device specific information (e.g., device model, device configuration, device characterizations, etc.). The model can include one or more of a machine learning model and an artificial intelligence model.

Once processing circuitry 82 has determined the suggested recharge interval value (e.g., based on the determined recharge interval), programmer 204 may generate, for output to a user, a suggested recharging habit. For example, the suggested habit can include an indication of the suggested recharge interval value. In general, the suggested habit includes an indication of when the user needs to recharge power source 18 of IMD 210, which may include an indication of the next recharge and other future recharges. In some examples, the suggested recharging habit (e.g., the indication of the suggested recharge interval value) can include one or more of an interval value (e.g., a period of time, such as 1 day, 3 weeks, 6 months, 1 year, etc.), a frequency value (e.g., daily, every 3 weeks, every 6 months, yearly, every Monday and Tuesday, etc.), a date (e.g., such as a date on a calendar, e.g., October 24, 2023), or another suitable indication of recharging information. Other examples of intervals used for suggested recharging habits can correspond to phases of celestial bodies (e.g., every full moon, every four full moons, and/or the like), relevant milestones of the patient or another person (e.g., birthdays, anniversaries, and/or the like), and/or other dates and/or periods of interest (holidays, seasons, and/or the like). Such suggested recharging habits described herein can facilitate fewer missed recharge sessions as well as reduce a likelihood of complete energy depletion of power source 18. Such suggested recharging habits described herein can facilitate reduced mental load on the patient (e.g., by reducing the mental load needed to remember to initiate recharging sessions). In some examples, the interval (e.g., the suggested recharge interval) may be automatically generated and presented for selection to the user based on user profile information (e.g., user-specific information that may include birthdays, etc.), and/or manually customized by a user via a user interface. In this manner, the system may generate a user interface that enables user selection of different recharge intervals (e.g., one or more suggested recharge intervals) as described herein.

In some examples, the indication of suggested recharge interval additionally or alternatively includes other indications, such as a countdown to next recharge, a calendar indicating one or more past or future recharges, or related information. In general, while this disclose is discussed primarily in the context of determining and generating suggested recharge interval values, processing circuitry 82 may be configured to generate any of the recharging information described herein (e.g., habits, frequencies, dates, prompts, etc.) using the similar techniques as described herein. For example, any instance of the recharging intervals and/or suggested recharging intervals, including suggested recharging values, can be understood to be interchangeable with any of the recharging information described herein (e.g., habits, frequencies, dates, prompts, etc.).

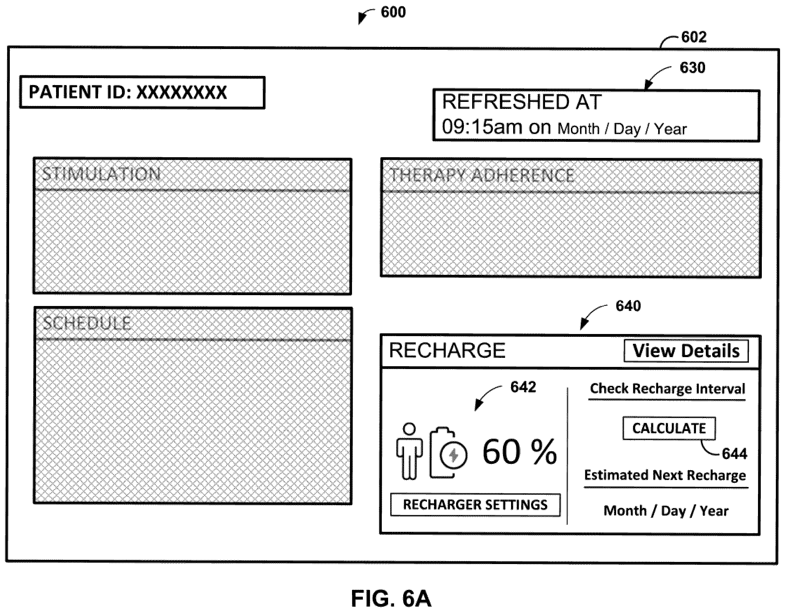

In some examples, programmer 204 generates, e.g., via processing circuitry 82, the indication of suggested recharge interval on user interface 86 of programmer 204. In some examples, programmer 204 generates, e.g., via processing circuitry 82, the indication of suggested recharge interval for presentation on another device (e.g., external charging device 208, a mobile computing device such as a phone or table, and/or the like), such as by communication via communication circuitry 88. In some examples, the indication of suggested recharge interval includes a visual display on a screen (e.g., as discussed in relation to FIGS. 6A-6G). In some examples, the indication of suggested recharge interval includes a prompt (e.g., a pop-up window, notification, etc.) on user interface 86, and/or another suitable user interface, indicating the suggested recharge interval. In some examples, the indication of suggested recharge interval includes an audible notification such as via text-to-voice, or another suitable audible notification (phone call, beeps, ringtone, etc.). In some examples, the indication of suggested recharge interval includes a haptic notification (e.g., a haptic notification via programmer 204, external charging device 208, and/or IMD 210), which may be a vibration or stimulation-based notification. Other suitable notifications are contemplated, including, but not limited to, text messages, electronic mail, push notifications, or other suitable notification to one or more devices.

In some examples, processing circuitry 82 of programmer 204 is configured to generate, for output, the indication of suggested recharge interval via external charging device 208. In some examples, external charging device 208 is configured to receive (e.g., via telemetry circuitry 56 and/or primary processing circuitry 50) the indication of suggested recharge interval, such as from programmer 204. External charging device 208 can be configured to generate, by primary processing circuitry 50 and for output, the indication of suggested recharge, such as via user interface 54.

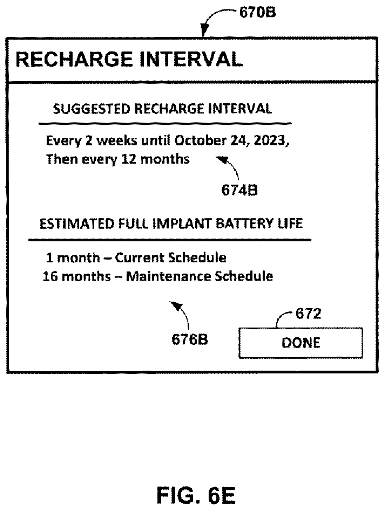

In some examples, processing circuitry 82 is configured to generate, for output to a user, multiple indications of suggested recharge interval values, such as for each therapy program of the at least one therapy program and/or for each therapy schedule of multiple therapy schedules. For example, as discussed in connection with at least FIG. 6E, the indication of the suggested recharge interval may include a first indication of a suggested recharge interval value for the first therapy program (e.g., which may include an induction schedule) and a second indication of a suggested recharge interval value for the second therapy program (e.g., which may include a maintenance schedule). In some examples, the first suggested recharge interval value is different than the second suggested recharge interval value, such that processing circuitry 82 generates different indications of suggested recharge interval values for each of the first therapy program and the second therapy program. However, in some examples, the first suggested recharge interval value and the second suggested recharge interval value are equal, and processing circuitry 82 generates only one indication of the suggested recharge interval.

In examples where processing circuitry 82 generates multiple indications of suggested recharge intervals (e.g., at least a first suggested recharge interval value and a second suggested recharge interval value) for different therapy schedules, processing circuitry 82 may also generate, for output, an indication of a transition between each of the multiple therapy schedules. While any number of therapy schedules is possible, the transition is described in connection between a first therapy schedule and a second therapy schedule. As illustrated in and discussed with respect to at least FIG. 6E, the indication of the transition between the first therapy schedules and the second therapy schedules may include one or more of a date (e.g., month, day, and year) of when the transition will occur, a countdown (e.g., an amount of time, including an amount of second, hours, minutes, days, weeks, months, etc.) until the transition will occur, or another suitable indicator of the transition between the first therapy schedule and the second therapy schedule. In some examples, processing circuitry 82 is configured to generate an indication of a transition between each therapy schedule of the multiple therapy schedules. In some examples, processing circuitry 82 generates an indication between a current therapy schedule and the next therapy schedule, e.g., even though more therapy schedules are programmed to occur after the next therapy schedule.

In some examples, processing circuitry 82 is configured to determine and generate, for output, other information related to the status of IMD 210, including a status of power source 18, as discussed in relation to previous examples. In some examples, processing circuitry 82 generates, for output, an indication of an amount or time remaining or an amount of charge remaining of power source 18 of IMD 210. In some examples, processing circuitry 82 is configured to determine and generate, for output, an indication of an amount of time before power source 18 drops below a threshold energy level (e.g., even if the amount of time does not correlate to the current energy level of power source 18). For example, processing circuitry 82 may be configured to determine and generate, for output, an indication of the amount of time power source will last on a full charge on a current and/or future therapy schedule.

In some examples, processing circuitry 82 generates, for output to a user, one or more prompts related to a status of power source 18 of IMD 210. In some examples, processing circuitry 82 generates, for output to a user, a prompt to recharge. In some examples, where processing circuitry 82 receives an indication that power source 18 has low energy (e.g., energy below a certain predefined threshold), such that power source 18 needs to be recharge immediately, processing circuitry 82 can generate a prompt to charge immediately (e.g., today).

In some examples, processing circuitry 82 generates a prompt to recharge based on the suggested recharge interval. For example, where the suggested recharge interval is weekly, processing circuitry 82 may generate one or more prompts to recharge at a regular weekly interval (which may be a selected by processing circuitry 82 or by the user). Processing circuitry 82 can be configured to generate a prompt, directed to a user, via external charging device 208 (e.g., via user interface 54 of external charging device 208).

In some examples, external charging device 208 is configured to execute one or more actions, generate information, and/or store information based on (e.g., in response to) the suggested recharge interval. For example, primary processing circuitry 50 of external charging device 208 can receive information of the suggested recharge interval (e.g., from programmer 204) and execute one or more actions, generate information, and/or store information based on (e.g., in response to) the information of suggested recharge interval. In some examples, primary processing circuitry 50 of external charging device 208 determines, based on information of suggested recharge interval, whether to generate a prompt or a notification to a user (e.g., a patient). For example, primary processing circuitry 50 can determine whether the next suggested recharging session is within a predefined time period (e.g., less than 1 day) and generate a prompt for a user to recharge IMD 210. In some examples, primary processing circuitry 50 of external charging device 208 automatically displays (e.g., via user interface 54) a prompt to recharge IMD 210. In some examples, primary processing circuitry 50 of external charging device 208 can switch external charging device 208 from a standby mode to a charging mode for recharging IMD 210 based on the suggested recharge interval (e.g., when a recharge session is due). For example, primary processing circuitry 50 of external charging device 208 can cause external charging device 208 to automatically (e.g., without user input) initiate recharging of power source 18 of IMD 210. Such functionalities of external charging device 208 can reduce a patient's reliance on programmer 204 and/or other devices (e.g., a mobile phone) for reminders and/or prompts to recharge power source 18 of IMD 210, which may facilitate reduced missed recharging sessions.

In some examples, processing circuitry 82 is configured to receive user input and determine, based on the user input, the suggested recharge interval. User input can include one or more of a user preference (e.g., patient preference) regarding recharging, information of a user's life style (e.g., sleep schedule, schedule of visits to a physician or other care provider, and the like), and/or a pattern of interactions with IMD 210, programmer 204, and/or external computing device 208. In some examples, processing circuitry 82 is configured to receive user input information (e.g., user preferences, user life style information, interaction pattern) and determine the suggested recharge interval based on the user input information as well as therapy parameters and/or the therapy schedule of each therapy program. For example, in some examples, processing circuitry 82 is configured to receive user input regarding information of scheduled visits to a clinic, e.g., such that processing circuitry 82 determines the suggested recharge interval to align with when a patient will be at the clinic for recharging of IMD 210 at the clinic.

In some examples, processing circuitry 82 is configured to receive an input of a user preference and determine, based on the user preference, the suggested recharge interval. For example, if a user has a preference to recharge at a particular reoccurring time and/or date (e.g., by dates on a calendar and/or by another interval, such as phase of the moon) in alignment with the suggested recharge interval, processing circuitry 82 may be configured to provide prompts to recharge in alignment with that user preference (e.g., if the suggested recharge interval is weekly and the user prefers to recharge on a particular day of the week, such as Sunday, processing circuitry 82 may be configured to receive input of the user preference to recharge every Sunday and generate prompts to recharge accordingly). A user preference may include a preference to recharge on a particular day of the week, a particular week of the month, a particular month of the year, a particular time of day, and/or a combination of any such preferences. A user preference may include a preference to avoid recharge, and/or prompts to recharge, on certain days and/or during certain times (e.g., such as on the weekends, during a planned vacation, and/or the like). Other examples of user preferences can include a preference to recharge when a celestial body is at a particular phase (e.g., every full moon, every four full moons, and/or the like), a preference to recharge on a particular relevant milestone (e.g., a birthday, an anniversary, a holiday, and/or the like), and/or a combination of any such preferences. Prompts to recharge may be configured to be delivered according to any of the previously described notification modalities without limitation, including pop-up window on a display, audible notification such as via text-to-voice, or another suitable audible notification (phone call, beeps, ringtone, etc.), a visual notification on a display, text messages, electronic mail, push notifications, or other suitable notification to one or more devices.

In some examples, processing circuitry 82 is configured to receive user input to override or change the system generated suggested recharge interval. For example, where a system-defined (e.g., determined by processing circuitry 82) suggested recharge interval is determined, processing circuitry 82 may receive input of a user-defined recharge interval and generate, for output, information according to the user-defined recharge intervals. Given that some users (e.g., patients, clinicians) may have a preference for relatively shorter or relatively longer recharging intervals, processing circuitry 82 may be configured to receive input of such preferences, such as for determining the suggested recharge intervals generated for presentation to the user. As such, in some examples, the user-defined recharge interval value is different than the plurality of predefined suggested possible recharge interval values. As an illustrative example, processing circuitry 82 may determine a system-defined suggested recharge interval of 2 weeks, but a user (e.g., a patient, clinician, etc.) would prefer to have a charging intervals of 1 week (and thus is more frequent than may otherwise be needed). In this example, processing circuitry 82 may be configured to receive input of this preference of a 1 week charging intervals and update the output of suggested recharge intervals and/or prompts of recharge intervals accordingly. In this way, processing circuitry 82 generates, for output based on the input of the user-defined recharge intervals, an indication of the user-defined recharge interval value. This functionality may increase the likelihood that a patient remembers to recharge, particularly in instances of long suggested recharge intervals (e.g., 6 months or more). In some examples, where a user-defined charging intervals is longer than the system-defined suggested recharge intervals, processing circuitry 82 may be configured to generate a warning and/or disallow input of a user-defined charging interval above (e.g., less frequent than) the suggested recharge interval. In this way, processing circuitry 82 may be configured to receive input of a user preference (e.g., patient preference, clinician preference, etc.), and determine one or more suggested recharge interval values based on the user preference. In some examples, user-defined charging intervals are programmed (e.g., by a user, such as a clinician) to align with scheduled visits to a clinic, e.g., for recharging at the clinic.

In some examples, processing circuitry 82 is configured to update (e.g., automatically and/or in response to user input) information related to the status of power source 18 of IMD 210 and/or suggested recharge intervals. In some examples, programmer 204, e.g., via communication circuitry 88, may communicate with IMD 210 and/or external computing device 208 to receive updated information. In some examples, processing circuitry 82 updates the determined recharge interval value by at least communicating with IMD 210 (e.g., which can include interrogating IMD 210). In some examples, processing circuitry 82 receives (e.g., from IMD 210, external charging device 208, and/or from another device) updated information for a therapy program (e.g., updated therapy parameters, information of therapy actually delivered to the patient, etc.). In some examples, programmer 204 is prompted to communicate with IMD 210 and/or external computing device 208 automatically at predefined time points (e.g., to ensure an accurate recharge interval value). In some example, programmer 204 is configured to receive user input prompting programmer 204 to communicate with IMD 210 and/or external computing device 208. Processing circuitry 82 may be configured to update information related to the status of power source 18 and/or suggested recharge intervals based on the communication with IMD 210 and/or external computing device 208. Processing circuitry 82 may be configured to update, based on the updated information for the therapy program, the determined recharge interval value.

In examples where processing circuitry 82 is configured to update information related to the status of power source 18 of IMD 210 and/or suggested recharge intervals, processing circuitry 82 may be configured to update information based on one or more trigger events. For example, trigger events may include detected changes in therapy programs, changes in therapy schedules, changes to therapy parameters, changes in therapy actually delivered to a user and/or deviations from predicted therapy delivery. Processing circuitry 82 may be configured to detect such trigger events, including instances of actual therapy delivery not in accordance with a therapy schedule (e.g., which may lead to more or less instances of therapy delivery as compared to a predefined therapy schedule). Processing circuitry 82 may be configured to detect trigger events including instances where therapy actually delivered to a patient was different than prescribed by the therapy program (e.g., such as instances where user ended a therapy delivery session sooner than the program-defined length of therapy delivery). Processing circuitry 82 may be configured to detect trigger events including instances where therapy actually delivered was different over a period time as compared to a therapy schedule (e.g., instances of skipped sessions). Accordingly, processing circuitry 82 may be configured to adjust and/or update the determined (e.g., calculated) recharge interval and/or the suggested recharge interval based on one or more trigger events, including changes to therapy programming and/or instances of actual therapy delivery. In some examples, processing circuitry 82 is configured to generate, for output to the user, a prompt or notification if and/or when information (such as the suggested recharge interval value) is updated and/or changed. In this way, a user may always be presented to the most accurate suggested recharge interval and/or system information, as well as know when information changes.

In some examples, processing circuitry 82 may be configured generate a default suggested recharge interval value. For example, prior to receiving information of therapy programming and/or one or more therapy schedules, processing circuitry 82 may be configured to generate, for display, a default suggested recharge interval value (e.g., 6 months), which may be a value that prevents power source 18 from dropping below a threshold level without delivering therapy. However, upon receiving information of therapy programming, processing circuitry 82 may be configured to update the default suggested recharge interval value to one or more different suggested recharge interval values according to the techniques described above.

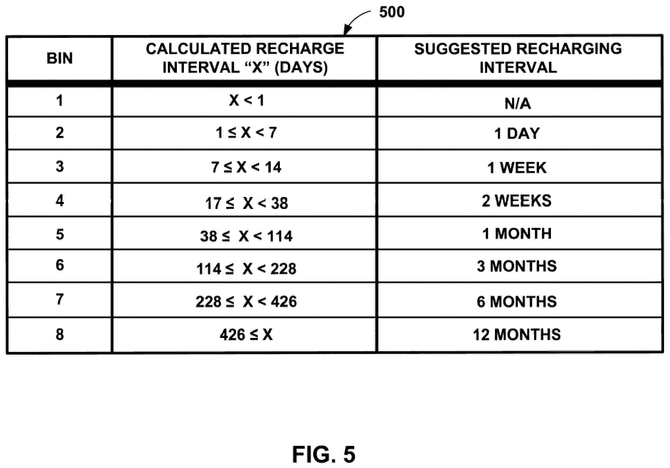

FIG. 5 includes a table 500 showing a plurality of bins (e.g., bin 1, bin 2, bin 3, bin 4, bin 5, bin 6, bin 7, bin 8), wherein each bin is defined in terms of determined (e.g., calculated) recharge interval values (e.g., expressed as a number "X" days) and corresponds to a suggested recharge interval. The bins and the corresponding suggested recharge interval values of FIG. 5 illustrate an example of how processing circuitry 82 of programmer 204 described in connection with FIG. 4 determines suggested recharge interval based on determined (e.g., calculated) recharge interval values. The suggested recharge interval values are relatively simple (e.g., whole numbers, integers, etc.) time periods that may be easier for a user to remember, and may align with periods typically occur on a user's schedule. The suggested recharge interval values shown in table 500 include 1 day (e.g. referred to as daily or every day as a frequency) corresponding to bin 2, 1 week (e.g., otherwise referred to as weekly as a frequency) corresponding to bin 3, 2 weeks (e.g., otherwise referred to as bi-weekly as a frequency) corresponding to bin 4, 1 month (e.g., otherwise referred to as monthly as a frequency) corresponding to bin 5, 3 months (e.g., otherwise referred to as quarterly as a frequency) corresponding to bin 6, 6 months (e.g., otherwise referred to as bi-annually as a frequency) corresponding to bin 7, and 12 months (e.g., otherwise referred to as yearly or annually as a frequency) corresponding to bin 8.

In some examples, one or more bins (e.g., bin 1 as shown in the example of FIG. 5) may be associated with no suggested recharge interval such as in examples where a calculated recharge interval is below a specified period of time (e.g., 1 day). In this example, a calculated recharge interval below the specified period of time may indicate a system error or programming error. Further, processing circuitry 82 may be configured to generate a prompt for a user (e.g., a clinician) to change programming when the calculated recharge interval is below the specified period of time, e.g., such that the suggested recharge interval would be too short (e.g., too frequent). In some examples, processing circuitry 82 may be configured to initiate an integrity test, such as to redetermine the recharge interval value when the calculated recharge interval is below the specified period of time.

The number (e.g., amount) of bins, the calculated recharge interval range associated with each bin, and the suggested recharge interval associated with each bin in table 500 is merely an illustrative example, and any suitable number of bins (e.g., one bin, two bins, three bins, four bins, five bins, six bins, seven bins, eight bins, nine bins, ten bins, fifteen bins, twenty bins, fifty bins, etc.), calculated recharge interval ranges, and suggested recharge interval values associated with each bin may be selected (e.g., programmed, changed, updated) and used as necessary. Suggested recharge interval values can include any suitable values, including suggested recharge interval values of a few minutes (e.g., 5 minutes, 10 minutes, 30 minutes), to one or more hours (e.g., 1 hour, 5, hours, 10 hours, etc.), to one or more days (e.g., every day, every 5 days, etc.), to one or more weeks (e.g., 1 week, 2 weeks, 3 weeks, etc.), to one or more months (e.g., 1 month, 2 months, 6 months, etc.), to one or more years (e.g., 1 year, 2 years, etc.). In some examples, the number (e.g., amount) of bins, the calculated recharge interval range associated with each bin, and the suggested recharge interval value associated with each bin are the same for different therapy schedules and therapy programs. In some examples, the number (e.g., amount) of bins, the calculated recharge interval range associated with each bin, and the suggested recharge interval value associated with each bin are different for different therapy schedules (e.g., different between a first therapy schedule and a second therapy schedule) and/or for different therapy programs (e.g., different between a first therapy program and a second therapy program). For example, a first therapy program, which may include a first therapy schedule, may be associated with a first set of bins, and a second therapy program, which may include a second therapy schedule different than the first therapy schedule, may be associated with a second set of bins. In this example, a number of bins of the first set of bins may be different than a number of bins of the second set of bins.

In some examples, at least some ranges of the plurality of respective ranges and/or bins include an upper bound value and/or a lower bound value. For example, in the example of FIG. 5, bin 1 only includes an upper bound value, indicating that any determined recharge interval value less than the particular upper bound correlates to bin 1. As another example, bin 8 only includes a lower bound value, indicating that any determined recharge interval value greater than or equal to the particular upper bound correlates to bin 8. However, each bin of bins 2-7 include both an upper bound value and a lower bound value.

In the example of table 500, bins 2-7 are defined such that calculated recharge interval values greater than or equal to the lower bound bin value and less than the upper bound bin value fall into (e.g., are associated with) the corresponding bin. Furthermore, lower bound values and upper bound values are defined as discrete values that are non-overlapping between bins. However, bins may be defined in other ways without limitation. For example, bins may be defined such that recharge interval values greater than the lower bound bin value and less than or equal to the upper bound bin value fall into the corresponding bin. As another example, upper bound values and lower bound values may overlap with other bins, e.g., such that a recharge interval value may fall into more than one bin. In these instances, processing circuitry 82 may prompt a user to select a preferred suggested recharge interval value from multiple valid suggested recharge interval values.

Further, bins, such as any of bins 2-7 may be defined differently between different therapy programs (e.g., between different therapy schedules). For example, upper bound and lower bound values may differ between different therapy programs. In some examples, a first set of bins associated with a first therapy schedule includes a first set of upper bound values and lower bound values for each bin in the first set of bins, and a second set of bins associated with a second therapy schedule includes a second set of upper bound values and lower bound values for each bin in the second set of bins. In some examples, the first set of upper bound values and lower bound values include different values than the second set of upper bound values and lower bound values. In some examples, the first set of upper bound values and lower bound values includes at least some of the same values as the second set of upper bound values and lower bound values.

In some examples, the number of bins, as well as upper bound values and/or lower bound values are dynamic (e.g., configured to be selected, changed, and/or updated at one or more time intervals). For example, a user (e.g., a clinician, a patient, a caretaker, etc.) may program the number of bins, and/or as upper bound values and/lower bound values. Processing circuitry 82 may be configured to receive input from a user selecting one or more of a number of bins, upper bound values, and/or lower bound values. In some examples, processing circuitry 82 may be configured to automatically determine (e.g., select, update, etc.) one or more of the number of bins, upper bound values, and/or lower bound values. In other examples, the number of bins, the upper bound values, and/or the lower bound values are predetermined and static, such that they are not able to be changed. In some examples, a user may program the suggested recharge intervals associated with some or all of the bins.

In some examples, the number of bins, upper bound values and/or lower bound values, as well as the suggested recharge interval associated with each bin may adjusted for different types of therapy, different patient conditions, different patient factors, or for other suitable reasons. For example, the number of bins, upper bound values and/or lower bound values, as well as the suggested recharge interval associated with each bin may correspond to a type of therapy (e.g., tibial stimulation for incontinence therapy, sacral neuromodulation, SCS, DBS) or a patient condition (e.g., urinary incontinence). For example, some more frequent types of stimulation therapy may require more frequent recharging, on the order of hours or days, and bins and suggested recharge interval values may be adjusted accordingly. In some examples, the number of bins, upper bound values and/or lower bound values, as well as the suggested recharge interval associated with each bin correspond to one or more patient factors such as patient age, disease status, patient gender, or another suitable patient factor. In some examples, suggested recharge intervals, bins, or other values are programmed (e.g., by a user, such as a clinician). For instances, the suggested recharge intervals, bins, or other values are programmed to align with scheduled visits to a clinic, e.g., for recharging at the clinic.

As discussed, the aforementioned technique of using bins to determine suggested recharge interval values may enable processing circuitry 82 (e.g., processing circuitry 82 of programmer 204 of FIG. 4) to select an appropriate suggested recharge interval from a plurality of predetermine recharging intervals. While described primarily in the context of processing circuitry 82 of programmer 204, another suitable device may additionally or alternatively be configured to determine (e.g., calculate) recharge intervals and determine suggested recharge intervals. For example, processing circuitry of another suitable device, such as primary processing circuitry 50 of external computing device 208 of FIG. 3, processing circuitry 30 of IMD 210, and/or remote (e.g., cloud-based) processing circuitry (such as server 112) may be configured to determine (e.g., calculate) recharge intervals and determine suggested recharge intervals according to the techniques of this disclosure. Once processing circuitry 82 has determined the suggested recharge intervals, processing circuitry 82 may be configured to generate, for output to a user, and indication of the suggested recharge interval. Although bins are described as one example of determining a suggested recharge interval value for subsequent recharging, other types of ranges or equations defining such ranges may be used in other examples.

FIGS. 6A-6G illustrate various screens from an example user interface in accordance with the techniques of this disclosure, and demonstrate the functionality prescribed to various components in relation to outputting information to a user as discussed in relation to previous examples. However, the following examples are merely illustrative examples, and the figures and corresponding descriptions presented herein are one of several ways of presenting information to a user. The features discussed herein may be presented in any permutation or combination without limitation.

FIG. 6A is a conceptual diagram illustrating an example user interface 600 related to a rechargeable battery according to this disclosure. User interface 600 may include a screen 602 with various boxes or areas that display relevant information related to a device, such as IMD 10 or IMD 210. User interface 600 may be an example of user interface 54 of external charging device 208 as shown in FIG. 3, user interface 86 of programmer 204 as shown in FIG. 4 (which may be a patient programmer or a clinician programmer), or another suitable device (including a mobile computing device, such as a smartphone or tablet). User interface 600 may provide a user with information about power source of a device, and will be discussed in connection with power source 18 of IMD 210 from FIG. 2. Although user interface 600 may have static boxes to display information related to power source 18, one or more pop-up windows or pop-up boxes, tabs, or other types of alerts are contemplated. In some examples, pop-up boxes include dynamic box or widgets that overlay at least part of the screen (e.g., screen 602).

In the example of FIG. 6A, user interface 600 provides a user (which may be a patient, a clinician, or another suitable user), with information related to the status of a power source. For example, screen 602 includes box 640 (e.g., a widget) showing information related to a status of power source 18 of IMD 210. Box 640 may include an indication of the status of power source 18 of IMD 210, including various charts, graphs, colors, numerical, or pictorial indications of the status of power source 18 of IMD 210. For example, box 640 includes a power source status indication 642 (represented as a numerical percentage of charge remaining). Power source status indication 642 may include one or more indications of a status of power source 18, such as an expected date (e.g., including month, day, and/or year) of expected depletion of power source 18 (e.g., depletion below a predefined level). In this way, processing circuitry 82 is configured to generate, for output, an indication of an amount or time remaining or an amount of charge remaining of power source 18 of IMD 210.

In the example of FIG. 6A, user interface 600 provides a user with information related to charging (e.g., recharging) power source 18. In some examples, box 640 includes a date and/or interval of an expected next recharge (e.g., when power source 18 of IMD 210 needs to be recharged). The date and/or interval of an expected next recharge presented may be in accordance with the determined (e.g., calculated) recharge interval and/or the suggested recharge interval. In this way, processing circuitry 82 is configured to generate, for output, information related to the status of power source 18 of IMD 210 and/or information related to the determined (e.g., calculated) recharge interval and/or the suggested recharge interval.

In some examples, screen 602 includes box 630, which may provide an indication of when the user and/or the device last "refreshed" or "updated" the determination of power source 18 (e.g., a battery status). In this way, while IMD 210 may automatically update indications of a status of power source 18 and/or information regarding determined (e.g., calculated) recharge intervals and/or the suggested recharge intervals, user interface 600 enables a user to "refresh" or "update" such determinations. In some examples, processing circuitry 82 is configured to repeat determination of determined (e.g., calculated) recharge intervals and/or the suggested recharge intervals, such as to account for changes performance of power source 18 over the course of the lifecycle of power source 18.

In some examples, processing circuitry 82 generates one or more notifications for presentation on screen 602 related to power source 18, such as periodic notifications or prompts for a user to recharge power source 18 of IMD 210. For example, in response to power source 18 dropping below a predefined threshold, screen 602 may include one or more prompts or reminders to recharge power source 18 of IMD 210. As discussed in relation to previous examples, processing circuitry 82 may be configured to generate prompts for charging in accordance with one or more suggested recharge intervals. In some examples, processing circuitry 82 may generate periodic notifications or prompts to recharge before the suggested recharge interval comes due (e.g., weekly before the suggested recharge interval).

In some examples, box 640 includes one or more options (e.g., buttons, toggles, or another suitable mechanism) for a user to access information related to power source 18. For example, box 640 includes a button 644 to prompt a calculation of the estimated recharge interval (which may be a time until recharge, or an expected date of battery recharge, as discussed in connection previous examples) and/or determination of one or more suggested recharge interval values, in accordance with the techniques described in this disclosure. In some examples, upon receiving user input to button 644, processing circuitry 82 may determine one or more determined recharge interval values and/or suggested recharge interval, but may also generate one or more intermediate pop-ups or screens for a user to confirm such determination and/or to present further information to a user.

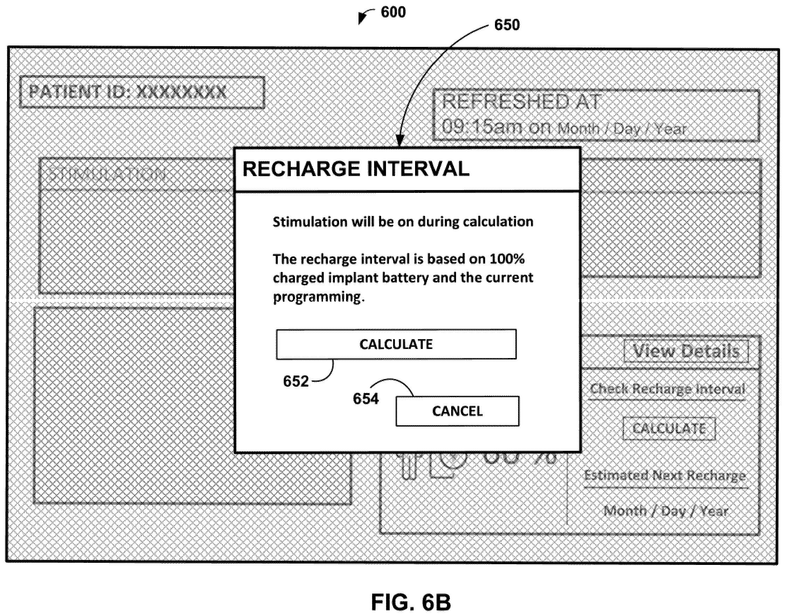

FIG. 6B is a conceptual diagram illustrating an example user interface, such as user interface 600 of FIG. 6A, which includes a pop-up box 650 generated by processing circuitry 82 as a result of receiving user input selecting button 644 in the example of FIG. 6A. In the example of FIG. 6B, pop-up box 650 includes information presented to a user as well as a button 652. As shown, pop-up box 650 may indicate to a user that stimulation will be "on" (e.g., activated) during determination (e.g., calculation) of determined recharge intervals and/or suggested recharge interval as well as other information pertinent to such determinations (e.g., that the determinations are based on a particular level of charge of the power source 18 of IMD 210 and/or based on current therapy programs, including current therapy settings and/or therapy schedules). Button 652 may prompt a user to confirm that determination (e.g., calculation) of determined recharge intervals and/or suggested recharge intervals will be initiated. In some examples, box 650 is not displayed, and processing circuitry 82 immediately initiates determination of the recharge interval and/or the suggested recharge intervals in response to receiving user input selecting button 644 in the example of FIG. 6A. In some examples, pop-up box 650 includes a cancel button 654, which enables a user to close pop-up box 650, which may terminate (or not initiate) determination of the recharge interval value and/or the suggested recharge interval values. For example, in response to receiving user input to button 654, processing circuitry 82 may be configured to return to screen 602 of FIG. 6A.

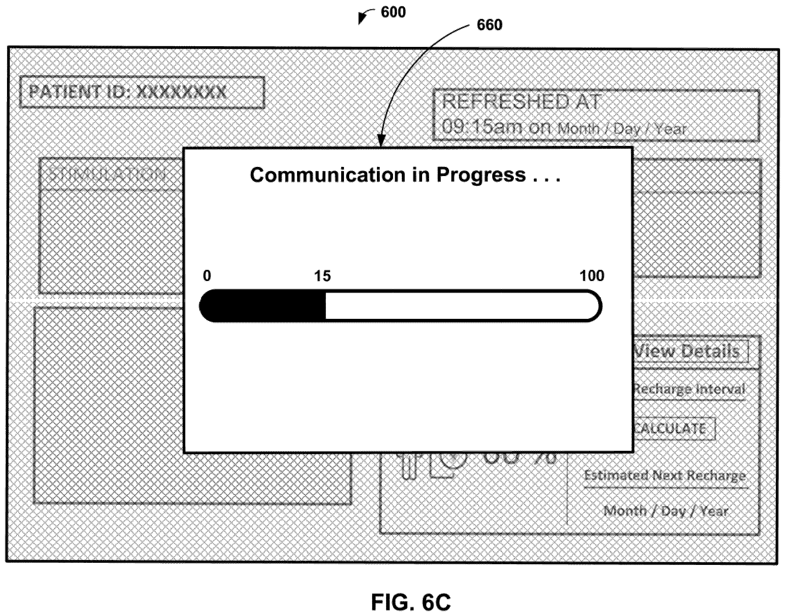

FIG. 6C is a conceptual diagram illustrating an example part of a user interface, such as user interface 600 of FIG. 6A, which includes a pop-up box 660 generated by processing circuitry 82 as a result of receiving user input selecting button 644 in the example of FIG. 6A and/or selecting button 652 in the example of FIG. 6B. Pop-up box 660 may provide an indication to a user of the status of the determination of the charging information by processing circuitry 82. For example, as shown in the example of FIG. 6C, pop-up box 660 displays a status bar indicating a status (e.g., progression) of the determination of charging information by processing circuitry 82. The status bar may also indicate various other steps, including communication with IMD 210. Pop-up box 660 may automatically clear, for example, when the progression bar is full and the determination of charging information is complete.

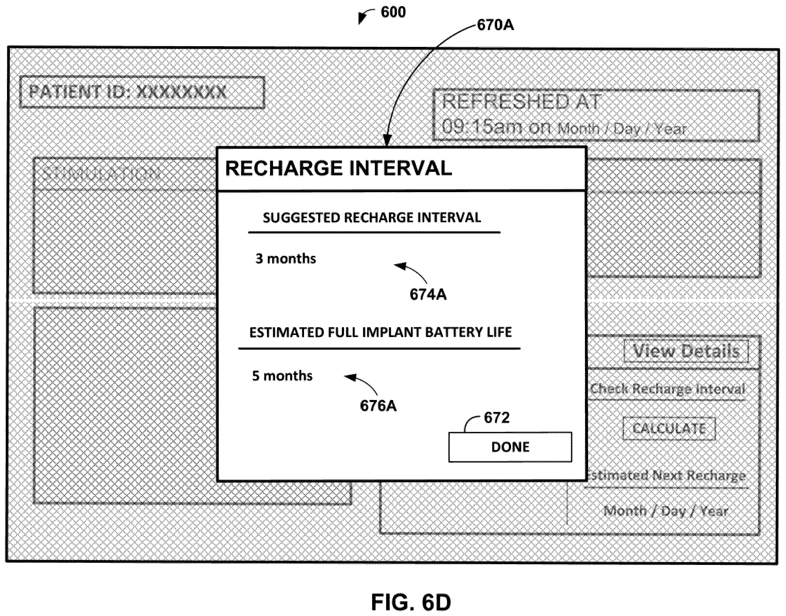

FIG. 6D is a conceptual diagram illustrating an example part of a user interface, such as user interface 600 of FIG. 6A, which includes a pop-up box 670A generated by processing circuitry 82 as a result of receiving user input selecting button 644 in the example of FIG. 6A and/or selecting button 652 in the example of FIG. 6B, and may come after pop-up box 660 of FIG. 6C.

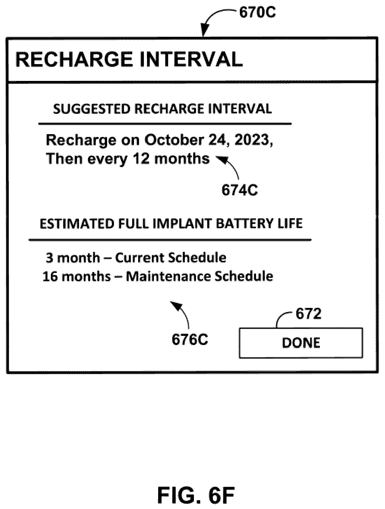

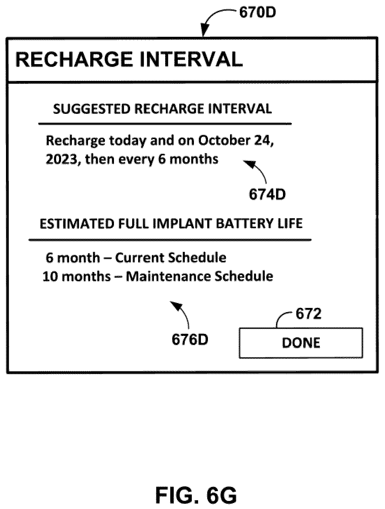

In the example of FIG. 6D, user interface 600 provides a user (which may be a patient, a clinician, or another suitable user), with information related to the status of power source 18 and/or information related to recharging power source 18. For example, the example of FIG. 6 shows pop-box 670A including an indication of a suggested recharge interval 674A (shown as "3 months" under the heading "Suggested Recharge Interval") and an indication of battery life 676A, which may include a determined (e.g., calculated) recharge interval (shown as "5 months" under the heading "Estimated Full Implant Battery Life"). As described herein, indication of battery life 676A may indicate a period of time until full depletion of power source 18 and/or a period of time for a power source 18 to drop below a threshold level (e.g., an operation level). In this way, processing circuitry 82 is configured to generate, for output to a user, an indication of a suggested recharge interval 674A (and/or a suggested recharging frequency). While the example of FIG. 6D illustrates indication of suggested recharge interval 674A and indication of the determined (e.g., calculated) recharge interval 676A as text, processing circuitry 82 may additionally or alternatively be configured to show one or more charts, graphs, pictorial, numerical or other indications of the suggested recharge interval and/or the determined recharge interval.

As discussed above, although this disclosure is discussed primarily in terms of intervals of time (e.g. interval values), which may represent a length or period of time (e.g., 1 week), it should be understood that frequencies (e.g., frequency values), which represent a recurring event (e.g., every day, every week, once per week, weekly, etc.) are contemplated to be used in addition to or instead of intervals, such as in any examples discussed herein where a system is configured to determine, use, and/or display such values in the context of recharging a power source of an IMD. For example, in the example of FIG. 6D, user interface 600 may display frequency values rather than interval values for recharging, for the indications of battery life, or other applicable values.

The manner in which processing circuitry 82 generates and displays indication of suggested recharge interval 674A and/or indication of the determined recharge interval 676A may be based on one or more of a number of therapy programs (e.g., more than one therapy program, including more than one therapy schedule), and a status of power source 18 (e.g., a level of power source 18 and/or whether power source 18 needs to be charged, such as before the transition between therapy schedules). In the example of pop-up box 670A of FIG. 6D, processing circuitry 82 generates a single value for indication of suggested recharge interval 674A, which may be applicable in examples where therapy programming includes only one therapy schedule, or the suggested recharge interval is equal for more than one therapy schedule. Similarly, processing circuitry 82 generates a single value for indication of the recharge interval 676A where therapy programming only includes one therapy schedule, or the recharge interval is equal for more than one therapy schedule.

In some examples, pop-up box 670A includes a button 672 which clears pop-up box 670A, which may cause user interface 600 to display (e.g., return to) screen 602 as shown in FIG. 6A. For example, in response to receiving user input selecting button 672, processing circuitry 82 may be configured to return to screen 602 of FIG. 6A.

FIGS. 6E, 6F, and 6G illustrate pop-up boxes 670B, 670C, and 670D respectively, which may be alternative examples of pop-up box 670A of FIG. 6D. Pop-up boxes 670B, 670C, and 670D may be generated instead of pop-up box 670A depending on a number of therapy programs (e.g., more than one therapy program, including more than one therapy schedule), and/or a status of power source 18 (e.g., a level of power source 18 and/or whether power source 18 needs to be charged, such as before the transition between therapy schedules). In examples including more than one therapy schedule, processing circuitry 82 may be configured to display one or more messages relating to the transition between the therapy schedules, including (but not limited to) a transition date between a first therapy schedule and a second therapy schedule, a prompt to charge on the date of the transition between the first therapy schedule and the second therapy schedule, or other messages related to the transition. While two therapy schedules are described for illustrative purposes, any number of therapy schedules are possible (e.g., one, two, three, four, five, six, seven, eight, nine, ten, fifteen, twenty or more therapy schedules). Additionally or alternatively, as shown in connection with FIG. 6G, processing circuitry 82 may generate, for display, an additional message to prompt a user to charge immediately (e.g., "today"), such when power source 18 is not above a predefined charge threshold (e.g., 90 percent). Processing circuitry 82 may be configured to generate for presentation any of pop-up boxes 670B, 670C, and 670D in addition to, or instead of, pop-up box 670A.

FIG. 6E, which illustrates pop-up box 670B, displays information in an example including at least a first therapy schedule (e.g., an induction schedule) and a second therapy schedule (e.g., a maintenance schedule), wherein the suggested recharge interval and the determined recharge interval for the first therapy schedule are different from the suggested recharge interval and the determined recharge interval for the second therapy schedule. Accordingly, pop-box 670B includes an indication of suggested recharge interval 674B including two different suggested recharge interval values (shown as "Every 2 weeks until October 24, 2023, Then every 12 months" under the heading "Suggested Recharge Interval") and an indication of battery life 676B, which may include one or more determined (e.g., calculated) recharge intervals. For example, indication of battery life 676B includes two different determined recharge interval values (shown as "1 month - current schedule" and "16 months - Maintenance Schedule" under the heading "Estimated Full Implant Battery Life"). In this way, processing circuitry 82 is configured to generate, for output to a user, indication of suggested recharge interval 674B and/or indication of the determined recharge interval 676B including multiple suggested recharge interval values and/or multiple determined recharge interval values based corresponding to multiple different therapy schedules (e.g., a first therapy schedule, such as "Current Schedule" and a second therapy schedule such as "Maintenance Schedule" in the example of FIG. 6E).

Pop-up box 670B also includes a transition date between the first therapy schedule and the second therapy schedule ("October 24, 2023"). In this way, processing circuitry 82 is configured to output an indication of a transition between the first therapy program and the second therapy program. In some examples, processing circuitry 82 is configured to determine a current date and compare the current date to the transition date, in which case processing circuitry may instead display pop-up box 670A in the example of FIG. 6D. For example, where a transition date between a first therapy program and a second therapy program (e.g., which may include a first therapy schedule and a second therapy schedule) has come to pass, processing circuitry may not display the transition date and/or may only display information relevant to the second therapy program.

FIG. 6F, which illustrates pop-up box 670C, is a variation of pop-up box 670B from FIG. 6E in which power source 18 does not need to be recharged before the transition date between the first therapy schedule and the second therapy schedule (e.g., power source 18 is charged above a predefined threshold and does not need to be charged before the transition date between the first therapy schedule and the second therapy schedule). For example, where a current date is sufficiently close to the transition date during the first therapy schedule, such that power source 18 does not need to be recharged, processing circuitry 82 does not generate for display a suggested recharge interval associated with the first therapy schedule. Rather, processing circuitry 82 only generates, for display, the suggested recharge interval value for the second therapy schedule. Accordingly, pop-box 670C includes an indication of suggested recharge interval 674C including one suggested recharge interval value corresponding to the second therapy schedule (shown as "Recharge on October 24, 2023, Then every 12 months" under the heading "Suggested Recharge Interval"). Pop-box 670C also includes an indication of battery life 676C, which may include one or more determined (e.g., calculated) recharge intervals. For example, indication of battery life 676C includes two different determined recharge interval values (shown as "3 months - current schedule" and "16 months - Maintenance Schedule" under the heading "Estimated Full Implant Battery Life").

FIG. 6G, which illustrates pop-up box 670D, is another variation of pop-up box 670B from FIG. 6E and/or pop-up box 670C of FIG. 6F. In the example of FIG. 6G, power source 18 needs to be charge before the transition date between the first therapy schedule and the second therapy schedule (e.g., because power source 18 is below the predetermined threshold), but if charged sufficiently, will not need to be recharged again until the transition date between the first therapy schedule and the second therapy schedule. Said another way, power source 18 only needs one more recharge session to last until the transition date between the first therapy schedule and the second therapy schedule in the example of FIG. 6G. In this way, processing circuitry 82 may generate for display pop-up box 670D when power source 18 should be recharged before the transition date between the first therapy schedule and the second therapy schedule, because power source 18 is not fully charged or otherwise not charged above a predefined threshold (e.g., not charge above a 90 percent of total charge capacity). Accordingly, pop-box 670D includes an indication of suggested recharge interval 674D including one suggested recharge interval value corresponding to the second therapy schedule, as well a prompt to "Recharge today" (shown as "Recharge today and on October 24, 2023, Then every 6 months" under the heading "Suggested Recharge Interval"). Pop-box 670D also includes an indication of battery life 676D which may include one or more determined (e.g., calculated) recharge intervals. For example, indication of battery life 676D includes two different determined recharge interval values (shown as "6 months - current schedule" and "10 months - Maintenance Schedule" under the heading "Estimated Full Implant Battery Life").

In general, once a transition (e.g., a transition date, as described in connection with pop-up box 670B, pop-up box 670C, and pop-up box 670D of FIG. 6E, FIG. 6F, and FIG. 6G respectively) between therapy schedules has come to pass, processing circuitry 82 may be configured to update information relating to the transition between therapy schedules. For example, processing circuitry 82 may be configured to generate information as if only one therapy schedule is programmed once the transition date between a first therapy schedule and second therapy schedule has come to pass. Such an example of information displayed for a single therapy schedule is shown and described in connection with FIG. 6D and pop-up box 670A.

The information shown described in connection with pop-up boxes pop-up box 670A, pop-up box 670B, pop-up box 670C, and pop-up box 670D of FIG. 6A FIG. 6E, FIG. 6F, and FIG. 6G respectively are merely illustrative examples, and may be displayed in any permutation or combination as appropriate. For example, processing circuitry 82 may be configured to generate a prompt to recharge immediately (e.g., "Recharge today") in any example where power source 18 is below a predefined threshold (e.g., including examples with one therapy schedule or more than one therapy schedule). Accordingly, examples like pop-up box 670A of FIG. 6D may include a prompt to recharge (e.g., "Recharge today"), which may be an example of one therapy schedule or where the suggested recharge interval for more than one therapy schedule is equal.

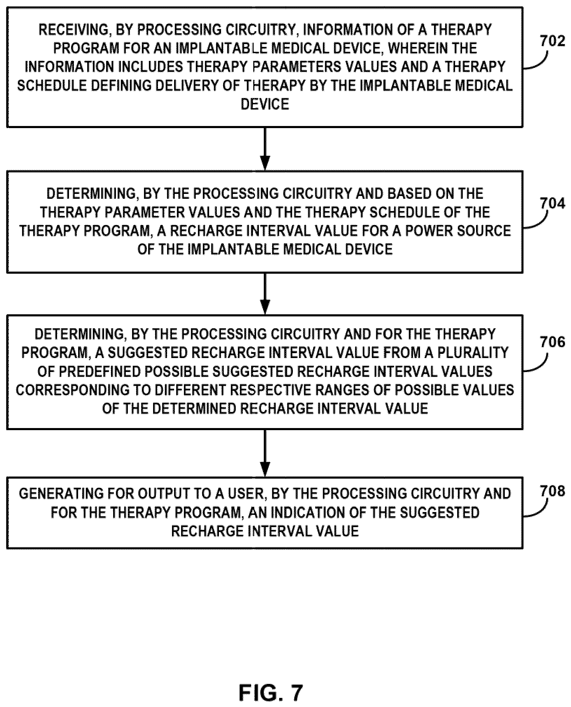

FIG. 7 is a flow chart illustrating an example technique for determining (e.g., calculating) a recharge interval of a power source of an IMD and generating a suggested recharge interval based on the determined (e.g., calculated) recharge interval. The example technique of FIG. 7 is discussed in relation to the components of IMD 210 as discussed in connection with FIG. 2, the components of external computing device 208 as discussed in connection with FIG. 3, and the components of programmer 204 as discussed in connection with FIG. 4, but may be used with any of the devices of this disclosure (e.g., IMD 10, external computing device 108, programmer 104, and/or server 112 of FIG. 1). While the steps described herein are described as being performed by processing circuitry 82 of programmer 204, the steps may also be performed by another device, alone or in combination with programmer 204. For example, one or more of primary processing circuitry 50 of external computing device 208, processing circuitry 30 of IMD 210, and/or remote (e.g., cloud-based) processing circuitry (such as server 112) may be configured to perform any of the steps discussed in relation to FIG. 7.

In the example of FIG. 7, processing circuitry 82 receives information of at least one therapy program for IMD 210 (702). As discussed in connection with previous examples, the information of each therapy program includes therapy parameters (e.g., amplitude, frequency, and/or pulse width) and a therapy schedule (e.g., predetermined schedules defining instances of therapy delivery) defining delivery of therapy by IMD 210. Processing circuitry 82 may receive information from IMD 210, such as by interrogated or communicating with IMD 210.

Processing circuitry 82 then determines, for each therapy program, a recharge interval value of power source 18 of IMD 210 based on the therapy parameters and the therapy schedule (704). The determined recharge interval value may indicate a period of time after which power source 18 the IMD 210 will run out of charge and/or drop below a predetermined level. The determined recharge interval may be based on a status (e.g., an instantaneous status or current status) of power source 18, as well as predicted future use according to the one or more therapy schedules. IMD 210 and/or processing circuitry 82 includes suitable components for determining recharge information for power source 18. In this way, processing circuitry 30 determines a suitable recharge interval such that IMD 210 delivers therapy according to a therapy schedule without running out of charge and/or without dropping below a threshold level of charge.

Processing circuitry 82 then determines, for each therapy program, a suggested recharge interval value from a plurality of predefined possible recharge interval values based on the determined recharge interval value (706). For example, processing circuitry 82 determines a suggested recharge interval value from a plurality of predefined possible suggested recharge interval values corresponding a respective range of possible values of the determined recharge interval value. In some examples, processing circuitry 82 determines the suggested recharge value which corresponds to one of a plurality of bins defined in terms of possible values of the recharge interval value. Each bin correlates a range of possible values of the determined recharge interval value to a suggested recharge interval value. To determine the suggested recharge interval value, processing circuitry 82 compares the determined recharge interval value to the range of the possible values of the determined recharge interval value for each bin of the plurality of bins. Based on the comparison, processing circuitry 82 determines (e.g., by selecting) the suggested recharge interval value corresponding to the bin in which the determined recharge interval value resides. In effect, processing circuitry 82 rounds the determined recharge interval value to the predetermined suggested recharge interval value associated with a particular bin.

Once processing circuitry 82 has determined the suggested recharge interval value for each therapy program, processing circuitry 82 then generates for output to a user, an indication of the suggested recharge interval (708). For example, as discussed in connection with FIGS. 6A-6G, processing circuitry 82 may generate for output to a user interface, information of the suggested recharge interval for each therapy program. The indication may include a first indication of a suggested recharge interval for a first therapy program (e.g., which may include an induction schedule) and a second indication of a suggested recharge interval for a second therapy program (which may include a maintenance schedule). The output may include one or more of a visual display on a screen (e.g., pop-up box, notification, etc.), and audible notification, a haptic notification, or another suitable notification. In some examples, the output includes a prompt on a user interface (e.g., user interface 86 of programmer 204) indicating the suggested recharge interval.

In some examples, processing circuitry 82 receives input of a user-defined recharge interval, which may include a user preference, and generates an indication of the user-defined recharge interval. For example, where a user has a preference to recharge more frequently than the system-defined suggested recharge interval, processing circuitry 82 may be configured to receive the user input indicating the user preference and generate the indication for output. For example, processing circuitry 82 may update the output of system-generated suggested recharge interval and/or prompts of recharge intervals accordingly based on the on the user-defined recharge interval.

In addition to generating an indication of the suggested recharge interval values for each therapy program, processing circuitry 82 may generate other information for output to a user. In some examples, processing circuitry 82 generates an indication of a transition (e.g., a transition date) between one or more therapy schedules. In some examples, processing circuitry 82 generates a prompt to recharge based on the suggested recharge interval. In some examples, processing circuitry 82 generates an indication of the suggested recharge interval for each therapy program (e.g., an indication of a recharge interval for an induction schedule and an indication of a recharge interval for a maintenance schedule). In some examples, processing circuitry 82 generates an indication of an amount or time remaining or an amount of charge remaining of power source 18 of IMD 210.

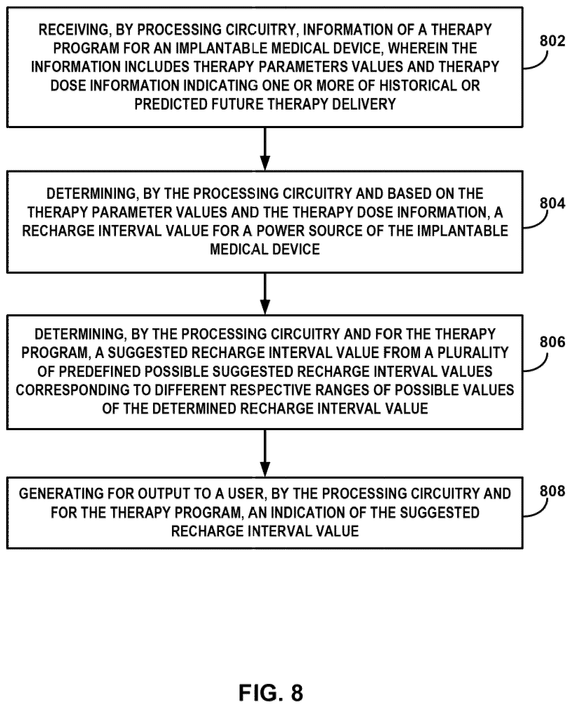

FIG. 8 is a flow chart illustrating an example technique for determining (e.g., calculating) a recharge interval of a power source of an IMD and generating a suggested recharge interval based on the determined (e.g., calculated) recharge interval. The example technique of FIG. 8 is discussed in relation to the components of IMD 210 as discussed in connection with FIG. 2, the components of external computing device 208 as discussed in connection with FIG. 3, and the components of programmer 204 as discussed in connection with FIG. 4, but may be used with any of the devices of this disclosure (e.g., IMD 10, external computing device 108, programmer 104, and/or server 112 of FIG. 1). While the steps described herein are described as being performed by processing circuitry 82 of programmer 204, the steps may also be performed by another device, alone or in combination with programmer 204. For example, one or more of primary processing circuitry 50 of external computing device 208, processing circuitry 30 of IMD 210, and/or remote (e.g., cloud-based) processing circuitry (such as server 112) may be configured to perform any of the steps discussed in relation to FIG. 8. FIG. 8 may include steps similar to the technique of FIG. 7, except as described herein.

In the example of FIG. 8, processing circuitry 82 receives information of at least one therapy program for IMD 210 (802). The information of each therapy program includes therapy parameters (e.g., amplitude, frequency, and/or pulse width) and therapy dose information indicating one or more of historical or predicted future therapy delivery.

Processing circuitry 82 then determines, for each therapy program, a recharge interval value of power source 18 of IMD 210 based on the therapy parameters and the therapy dose information (804).

As discussed in connection with FIG. 7, processing circuitry 82 then determines, for each therapy program, a suggested recharge interval value from a plurality of predefined possible recharge interval values based on the determined recharge interval value (806). Once processing circuitry 82 has determined the suggested recharge interval value for each therapy program, processing circuitry 82 then generates for output to a user, an indication of the suggested recharge interval (808).

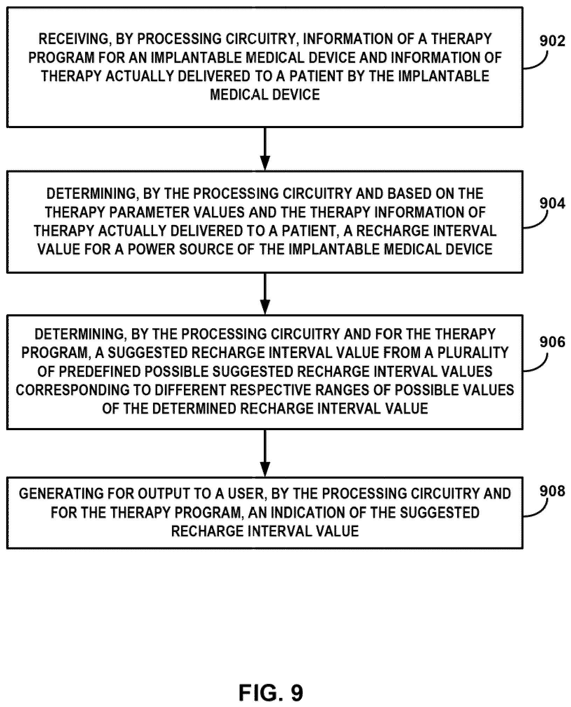

FIG. 9 is a flow chart illustrating an example technique for determining (e.g., calculating) a recharge interval of a power source of an IMD and generating a suggested recharge interval based on the determined (e.g., calculated) recharge interval. The example technique of FIG. 9 is discussed in relation to the components of IMD 210 as discussed in connection with FIG. 2, the components of external computing device 208 as discussed in connection with FIG. 3, and the components of programmer 204 as discussed in connection with FIG. 4, but may be used with any of the devices of this disclosure (e.g., IMD 10, external computing device 108, programmer 104, and/or server 112 of FIG. 1). While the steps described herein are described as being performed by processing circuitry 82 of programmer 204, the steps may also be performed by another device, alone or in combination with programmer 204. For example, one or more of primary processing circuitry 50 of external computing device 208, processing circuitry 30 of IMD 210, and/or remote (e.g., cloud-based) processing circuitry (such as server 112) may be configured to perform any of the steps discussed in relation to FIG. 9. FIG. 9 may include steps similar to the technique of FIG. 7, and FIG. 8, except as described herein.

In the example of FIG. 9, processing circuitry 82 receives information of a therapy program for IMD 210 and information of therapy actually delivered to a patient by the IMD 210 (902). The information of each therapy program includes therapy parameters (e.g., amplitude, frequency, and/or pulse width). In some examples, processing circuitry 82 determines therapy parameters based on physiological sensing via one or more sensors 37 (e.g., as part of a closed-loop therapy system). The information of therapy actually delivered to the patient by IMD 210 can include a record of one or more instances of therapy actually delivered to a patient.

Processing circuitry 82 then determines, for each therapy program, a recharge interval value of power source 18 of IMD 210 based on the therapy parameters and information of therapy actually delivered to a patient (904).

As discussed in connection with FIG. 7 and FIG. 8, processing circuitry 82 then determines, for each therapy program, a suggested recharge interval value from a plurality of predefined possible recharge interval values based on the determined recharge interval value (906). Once processing circuitry 82 has determined the suggested recharge interval value for each therapy program, processing circuitry 82 then generates for output to a user, an indication of the suggested recharge interval (908).

The techniques described in this disclosure may be implemented, at least in part, in hardware, software, firmware or any combination thereof. For example, various aspects of the described techniques may be implemented within one or more processors, such as fixed function processing circuitry and/or programmable processing circuitry, including one or more microprocessors, digital signal processors (DSPs), application specific integrated circuits (ASICs), field programmable gate arrays (FPGAs), or any other equivalent integrated or discrete logic circuitry, as well as any combinations of such components. The term "processor" or "processing circuitry" may generally refer to any of the foregoing logic circuitry, alone or in combination with other logic circuitry, or any other equivalent circuitry. A control unit comprising hardware may also perform one or more of the techniques of this disclosure.

Such hardware, software, and firmware may be implemented within the same device or within separate devices to support the various operations and functions described in this disclosure. In addition, any of the described units, modules or components may be implemented together or separately as discrete but interoperable logic devices. Depiction of different features as modules or units is intended to highlight different functional aspects and does not necessarily imply that such modules or units must be realized by separate hardware or software components. Rather, functionality associated with one or more modules or units may be performed by separate hardware or software components, or integrated within common or separate hardware or software components.

## Claims

1. A system (100) comprising:
processing circuitry (30) configured to:
receive information of a therapy program for an implantable medical device, (10) wherein the information includes therapy parameters values and a therapy schedule defining delivery of therapy by the implantable medical device, determine, based on the therapy parameter values and the therapy schedule of the therapy program, a recharge interval value for a power source (18) of the implantable medical device,
determine, for the therapy program, a suggested recharge interval value from a plurality of predefined possible suggested recharge interval values corresponding to different respective ranges of possible values of the determined recharge interval value, and
generate, for output to a user and for the therapy program, an indication of the suggested recharge interval value.

2. The system of claim 1,
wherein each bin of a plurality of bins correlates each range of the different respective ranges of the possible values of the determined recharge interval value to the plurality of predefined possible suggested recharge interval values, and
wherein to determine the suggested recharge interval value, the processing circuitry is configured to:
compare the determined recharge interval value to the range of the possible values of the determined recharge interval value for each bin of the plurality of bins, and
based on the comparison, select the suggested recharge interval value from the plurality of predefined possible suggested recharge interval values that corresponds to the bin in which the determined recharge interval value resides.

3. The system of any of the proceeding claims, wherein the therapy program comprises a first therapy program and a second therapy program, and wherein the indication of the suggested recharge interval value includes a first indication for the first therapy program and a second indication for the second therapy program, the first indication different than the second indication.

4. The system of claim 3, wherein the processing circuitry is configured to output an indication of a transition between the first therapy program and the second therapy program.

5. The system of any of the claims 3 or 4, wherein the first therapy program includes a first therapy schedule defining delivery of therapy by the implantable medical device and the second therapy program includes a second therapy schedule defining delivery of therapy by the implantable medical device, the first therapy schedule different than the second therapy schedule.

6. The system of any of the proceeding claims,
wherein at least some ranges of the possible values of the determined recharge interval value include an upper bound value and a lower bound value, and
wherein the suggested recharge interval value correlating to each range of the at least some ranges is lower than the lower bound value for the at least some ranges of the respective ranges.

7. The system of any of the proceeding claims, wherein the processing circuitry is configured to:
receive input of a user-defined recharge interval value different than the plurality of predefined suggested possible recharge interval values, and
generate, for output based on the input of the user-defined recharge interval value, an indication of the user-defined recharge interval value.

8. The system of any of the proceeding claims, wherein the processing circuitry is configured to:
automatically or in response to user input, receive updated information for the therapy program via communication with the implantable medical device; and
update, based on the updated information, the determined recharge interval value.

9. The system of any of the proceeding claims, wherein the processing circuitry is configured to generate, for output, a prompt on a user interface of a computing device indicating the suggested recharge interval value.

10. The system of any of the proceeding claims, wherein the processing circuitry is configured to control the implantable medical device to deliver electrical stimulation therapy to one or more of a sacral nerve or tibial nerve for incontinence therapy according to the therapy program.

11. A non-transitory computer-readable storage medium storing instructions that, when executed, cause processing circuitry (30) to perform a method, the method comprising:
receiving, by processing circuitry, information of a therapy program for an implantable medical device, (10)
wherein the information includes therapy parameters values and a therapy schedule defining delivery of therapy by the implantable medical device;
determining, by the processing circuitry and based on the therapy parameter values and the therapy schedule of the therapy program, a recharge interval value for a power source (18) of the implantable medical device;
determining, by the processing circuitry and for the therapy program, a suggested recharge interval value from a plurality of predefined possible suggested recharge interval values corresponding to different respective ranges of possible values of the determined recharge interval value; and
generating for output to a user, by the processing circuitry and for the therapy program, an indication of the suggested recharge interval value.

12. The non-transitory computer-readable storage medium of claim 11, wherein each bin of a plurality of bins correlates to each range of the different respective ranges of the possible values of the determined recharge interval value to the plurality of predefined possible suggested recharge interval values, and
wherein determining the suggested recharge interval value comprises:
comparing, by the processing circuitry, the determined recharge interval value to the range of the possible values of the determined recharge interval value for each bin of the plurality of bins, and
selecting, by the processing circuitry and based on the comparison, the suggested recharge interval value from the plurality of predefined possible suggested recharge interval values that corresponds to the bin in which the determined recharge interval value resides.

13. The non-transitory computer-readable storage medium of any of the claims 11 or 12, where the therapy program comprises a first therapy program and a second therapy program, and wherein the indication of the suggested recharge interval value includes a first indication for the first therapy program and a second indication for the second therapy program, the first indication different than the second indication.

14. The non-transitory computer-readable storage medium of any of claim 13, wherein the method further comprises:
outputting, by the processing circuitry, an indication of a transition between the first therapy program and the second therapy program.

## Patentansprüche

1. System (100), umfassend:
eine Verarbeitungsschaltung (30), die konfiguriert ist zum:
Empfangen von Informationen eines Therapieprogramms für eine implantierbare medizinische Vorrichtung, (10) wobei die Informationen Therapieparameterwerte und einen Therapieplan umfassen, der eine Abgabe von Therapie durch die implantierbare medizinische Vorrichtung definiert,
Bestimmen, basierend auf den Therapieparameterwerten und dem Therapieplan des Therapieprogramms, eines Wiederaufladeintervallwerts für eine Leistungsquelle (18) der implantierbaren medizinischen Vorrichtung,
Bestimmen, für das Therapieprogramm, eines vorgeschlagenen Wiederaufladeintervallwerts aus einer Vielzahl von vordefinierten möglichen vorgeschlagenen Wiederaufladeintervallwerten, die unterschiedlichen jeweiligen Bereichen von möglichen Werten des bestimmten Wiederaufladeintervallwerts entsprechen, und
Erzeugen, für die Ausgabe an einen Benutzer und für das Therapieprogramm, einer Angabe des vorgeschlagenen Wiederaufladeintervallwerts.

2. System nach Anspruch 1,
wobei jeder Bin einer Vielzahl von Bins jeden Bereich der unterschiedlichen jeweiligen Bereiche der möglichen Werte des bestimmten Wiederaufladeintervallwerts mit der Vielzahl von vordefinierten möglichen vorgeschlagenen Wiederaufladeintervallwerten korreliert, und
wobei, um den vorgeschlagenen Wiederaufladeintervallwert zu bestimmen, die Verarbeitungsschaltung konfiguriert ist zum:
Vergleichen des bestimmten Wiederaufladeintervallwerts mit dem Bereich der möglichen Werte des bestimmten Wiederaufladeintervallwerts für jeden Bin der Vielzahl von Bins, und
basierend auf dem Vergleich, Auswählen des vorgeschlagenen Wiederaufladeintervallwerts aus der Vielzahl von vordefinierten möglichen vorgeschlagenen Wiederaufladeintervallwerten, der dem Bin entspricht, in dem der bestimmte Wiederaufladeintervallwert verweilt.

3. System nach einem der vorstehenden Ansprüche, wobei das Therapieprogramm ein erstes Therapieprogramm und ein zweites Therapieprogramm umfasst, und wobei die Angabe des vorgeschlagenen Wiederaufladeintervallwerts eine erste Angabe für das erste Therapieprogramm und eine zweite Angabe für das zweite Therapieprogramm einschließt, wobei sich die erste Angabe von der zweiten Angabe unterscheidet.

4. System nach Anspruch 3, wobei die Verarbeitungsschaltung konfiguriert ist, um eine Angabe eines Übergangs zwischen dem ersten Therapieprogramm und dem zweiten Therapieprogramm auszugeben.

5. System nach einem der Ansprüche 3 oder 4, wobei das erste Therapieprogramm einen ersten Therapieplan umfasst, der die Abgabe von Therapie durch die implantierbare medizinische Vorrichtung definiert, und das zweite Therapieprogramm einen zweiten Therapieplan umfasst, der die Abgabe von Therapie durch die implantierbare medizinische Vorrichtung definiert, wobei sich der erste Therapieplan von dem zweiten Therapieplan unterscheidet.

6. System nach einem der vorstehenden Ansprüche,
wobei mindestens einige Bereiche der möglichen Werte des bestimmten Wiederaufladeintervallwerts einen oberen Grenzwert und einen unteren Grenzwert einschließen, und
wobei der vorgeschlagene Wiederaufladeintervallwert, der mit jedem Bereich der mindestens einigen Bereiche korreliert, niedriger als der untere Grenzwert für die mindestens einigen Bereiche der jeweiligen Bereiche ist.

7. System nach einem der vorstehenden Ansprüche, wobei die Verarbeitungsschaltung konfiguriert ist zum:
Empfangen einer Eingabe eines benutzerdefinierten Wiederaufladeintervallwerts, der sich von der Vielzahl von vordefinierten vorgeschlagenen möglichen Wiederaufladeintervallwerten unterscheidet, und
Erzeugen, für die Ausgabe basierend auf der Eingabe des benutzerdefinierten Wiederaufladeintervallwerts, einer Angabe des benutzerdefinierten Wiederaufladeintervallwerts.

8. System nach einem der vorstehenden Ansprüche, wobei die Verarbeitungsschaltung konfiguriert ist zum:
automatisch oder als Reaktion auf eine Benutzereingabe, Empfangen aktualisierter Informationen für das Therapieprogramm über eine Kommunikation mit der implantierbaren medizinischen Vorrichtung; und
Aktualisieren, basierend auf den aktualisierten Informationen, des bestimmten Wiederaufladeintervallwerts.

9. System nach einem der vorstehenden Ansprüche, wobei die Verarbeitungsschaltung konfiguriert ist, um für die Ausgabe eine Aufforderung auf einer Benutzerschnittstelle einer Rechenvorrichtung zu erzeugen, die den vorgeschlagenen Wiederaufladeintervallwert angibt.

10. System nach einem der vorstehenden Ansprüche, wobei die Verarbeitungsschaltung konfiguriert ist, um die implantierbare medizinische Vorrichtung zu steuern, um eine Elektrostimulationstherapie an einen oder mehrere von einem Sakralnerv oder Tibialnerv für eine Inkontinenztherapie gemäß dem Therapieprogramm abzugeben.

11. Nichtflüchtiges computerlesbares Speicherungsmedium, das Anweisungen speichert, die, wenn sie ausgeführt werden, die Verarbeitungsschaltung (30) veranlassen, ein Verfahren durchzuführen, das Verfahren umfassend:
Empfangen, durch eine Verarbeitungsschaltung, von Informationen eines Therapieprogramms für eine implantierbare medizinische Vorrichtung, (10) wobei die Informationen Therapieparameterwerte und einen Therapieplan umfassen, der die Abgabe von Therapie durch die implantierbare medizinische Vorrichtung definiert;
Bestimmen, durch die Verarbeitungsschaltung und basierend auf den Therapieparameterwerten und dem Therapieplan des Therapieprogramms, eines Wiederaufladeintervallwerts für eine Leistungsquelle (18) der implantierbaren medizinischen Vorrichtung;
Bestimmen, durch die Verarbeitungsschaltung und für das Therapieprogramm, eines vorgeschlagenen Wiederaufladeintervallwerts aus einer Vielzahl von vordefinierten möglichen vorgeschlagenen Wiederaufladeintervallwerten, die unterschiedlichen jeweiligen Bereichen von möglichen Werten des bestimmten Wiederaufladeintervallwerts entsprechen; und
Erzeugen, für die Ausgabe an einen Benutzer, durch die Verarbeitungsschaltung und für das Therapieprogramm, einer Angabe des vorgeschlagenen Wiederaufladeintervallwerts.

12. Nichtflüchtiges computerlesbares Speicherungsmedium nach Anspruch 11, wobei jeder Bin einer Vielzahl von Bins mit jedem Bereich der unterschiedlichen jeweiligen Bereiche der möglichen Werte des bestimmten Wiederaufladeintervallwerts mit der Vielzahl von vordefinierten möglichen vorgeschlagenen Wiederaufladeintervallwerten korreliert, und
wobei das Bestimmen des vorgeschlagenen Wiederaufladeintervallwerts umfasst:
Vergleichen, durch die Verarbeitungsschaltung, des bestimmten Wiederaufladeintervallwerts mit dem Bereich der möglichen Werte des bestimmten Wiederaufladeintervallwerts für jeden Bin der Vielzahl von Bins, und
Auswählen, durch die Verarbeitungsschaltung und basierend auf dem Vergleich, des vorgeschlagenen Wiederaufladeintervallwerts aus der Vielzahl von vordefinierten möglichen vorgeschlagenen Wiederaufladeintervallwerten, der dem Bin entspricht, in dem der ermittelte Wiederaufladeintervallwert verweilt.

13. Nichtflüchtiges computerlesbares Speicherungsmedium nach einem der Ansprüche 11 oder 12, wobei das Therapieprogramm ein erstes Therapieprogramm und ein zweites Therapieprogramm umfasst, und wobei die Angabe des vorgeschlagenen Wiederaufladeintervallwerts eine erste Angabe für das erste Therapieprogramm und eine zweite Angabe für das zweite Therapieprogramm einschließt, wobei sich die erste Angabe von der zweiten Angabe unterscheidet.

14. Nichtflüchtiges computerlesbares Speicherungsmedium nach einem der Ansprüche 13, wobei das Verfahren ferner umfasst:
Ausgeben, durch die Verarbeitungsschaltung, einer Angabe eines Übergangs zwischen dem ersten Therapieprogramm und dem zweiten Therapieprogramm.

## Revendications

1. Système (100) comprenant :
une circuiterie de traitement (30) configurée pour :
recevoir des informations concernant un programme thérapeutique pour un dispositif médical implantable (10), dans lequel les informations comportent des valeurs de paramètres thérapeutiques et un calendrier thérapeutique définissant l'administration d'une thérapie par le dispositif médical implantable,
déterminer, sur la base des valeurs de paramètres thérapeutiques et du calendrier thérapeutique du programme thérapeutique, une valeur d'intervalle de recharge pour une source d'alimentation (18) du dispositif médical implantable,
déterminer, pour le programme thérapeutique, une valeur d'intervalle de recharge suggérée parmi une pluralité de valeurs d'intervalle de recharge suggérées possibles prédéfinies correspondant à différentes plages respectives de valeurs possibles de la valeur d'intervalle de recharge déterminée, et
générer, pour une sortie à un utilisateur et pour le programme thérapeutique, une indication de la valeur d'intervalle de recharge suggérée.

2. Système selon la revendication 1,
dans lequel chaque casier d'une pluralité de casiers établit une corrélation entre chaque plage des différentes plages respectives des valeurs possibles de la valeur d'intervalle de recharge déterminée et la pluralité de valeurs d'intervalle de recharge suggérées possibles prédéfinies, et
dans lequel, pour déterminer la valeur d'intervalle de recharge suggérée, la circuiterie de traitement est configurée pour :
comparer la valeur d'intervalle de recharge déterminée avec la plage des valeurs possibles de la valeur d'intervalle de recharge déterminée pour chaque casier de la pluralité de casiers, et
sélectionner, sur la base de la comparaison, la valeur d'intervalle de recharge suggérée parmi la pluralité de valeurs d'intervalle de recharge suggérées possibles prédéfinies qui correspondent au casier dans lequel se trouve la valeur d'intervalle de recharge déterminée.

3. Système selon l'une quelconque des revendications précédentes, dans lequel le programme thérapeutique comprend un premier programme thérapeutique et un second programme thérapeutique, et dans lequel l'indication de la valeur d'intervalle de recharge suggérée comporte une première indication pour le premier programme thérapeutique et une seconde indication pour le second programme thérapeutique, la première indication étant différente de la seconde indication.

4. Système selon la revendication 3, dans lequel la circuiterie de traitement est configurée pour délivrer en sortie une indication d'une transition entre le premier programme thérapeutique et le second programme thérapeutique.

5. Système selon l'une quelconque des revendications 3 ou 4, dans lequel le premier programme thérapeutique comporte un premier calendrier thérapeutique définissant l'administration d'une thérapie par le dispositif médical implantable et le second programme thérapeutique comporte un second calendrier thérapeutique définissant l'administration d'une thérapie par le dispositif médical implantable, le premier calendrier thérapeutique étant différent du second calendrier thérapeutique.

6. Système selon l'une quelconque des revendications précédentes,
dans lequel au moins certaines plages des valeurs possibles de la valeur d'intervalle de recharge déterminée comportent une valeur de limite supérieure et une valeur de limite inférieure, et
dans lequel la valeur d'intervalle de recharge suggérée corrélée à chaque plage des au moins certaines plages est inférieure à la valeur de limite inférieure pour les au moins certaines plages des plages respectives.

7. Système selon l'une quelconque des revendications précédentes, dans lequel la circuiterie de traitement est configurée pour :
recevoir une entrée d'une valeur d'intervalle de recharge définie par utilisateur différente de la pluralité de valeurs d'intervalle de recharge possibles suggérées prédéfinies, et
générer, pour une sortie sur la base de l'entrée de la valeur d'intervalle de recharge définie par utilisateur, une indication de la valeur d'intervalle de recharge définie par utilisateur.

8. Système selon l'une quelconque des revendications précédentes, dans lequel la circuiterie de traitement est configurée pour :
recevoir, automatiquement ou en réponse à une entrée utilisateur, des informations mises à jour pour le programme thérapeutique par l'intermédiaire d'une communication avec le dispositif médical implantable ; et
mettre à jour, sur la base des informations mises à jour, la valeur d'intervalle de recharge déterminée.

9. Système selon l'une quelconque des revendications précédentes, dans lequel la circuiterie de traitement est configurée pour générer, pour une sortie, une invite sur une interface utilisateur d'un dispositif informatique indiquant la valeur d'intervalle de recharge suggérée.

10. Système selon l'une quelconque des revendications précédentes, dans lequel la circuiterie de traitement est configurée pour commander le dispositif médical implantable afin d'administrer une thérapie par stimulation électrique à un ou plusieurs parmi un nerf sacré ou un nerf tibial pour une thérapie d'incontinence selon le programme thérapeutique.

11. Support de stockage non transitoire lisible par ordinateur stockant des instructions qui, lorsqu'exécutées, amènent une circuiterie de traitement (30) à mettre en œuvre un procédé, le procédé comprenant :
la réception, par la circuiterie de traitement, d'informations concernant un programme thérapeutique pour un dispositif médical implantable (10), dans lequel les informations comportent des valeurs de paramètres thérapeutiques et un calendrier thérapeutique définissant l'administration d'une thérapie par le dispositif médical implantable ;
la détermination, par la circuiterie de traitement et sur la base des valeurs de paramètres thérapeutiques et du calendrier thérapeutique du programme thérapeutique, d'une valeur d'intervalle de recharge pour une source d'alimentation (18) du dispositif médical implantable ;
la détermination, par la circuiterie de traitement et pour le programme thérapeutique, d'une valeur d'intervalle de recharge suggérée parmi une pluralité de valeurs d'intervalle de recharge suggérées possibles prédéfinies correspondant à différentes plages respectives de valeurs possibles de la valeur d'intervalle de recharge déterminée ; et
la génération, pour une sortie à un utilisateur, par la circuiterie de traitement et pour le programme thérapeutique, d'une indication de la valeur d'intervalle de recharge suggérée.

12. Support de stockage non transitoire lisible par ordinateur selon la revendication 11, dans lequel chaque casier d'une pluralité de casiers établit une corrélation entre chaque plage des différentes plages respectives des valeurs possibles de la valeur d'intervalle de recharge déterminée et la pluralité de valeurs d'intervalle de recharge suggérées possibles prédéfinies, et
dans lequel la détermination de la valeur d'intervalle de recharge suggérée comprend :
la comparaison, par la circuiterie de traitement, de la valeur d'intervalle de recharge déterminée avec la plage des valeurs possibles de la valeur d'intervalle de recharge déterminée pour chaque casier de la pluralité de casiers, et
la sélection, par la circuiterie de traitement et sur la base de la comparaison, de la valeur d'intervalle de recharge suggérée parmi la pluralité de valeurs d'intervalle de recharge suggérées possibles prédéfinies qui correspondent au casier dans lequel se trouve la valeur d'intervalle de recharge déterminée.

13. Support de stockage non transitoire lisible par ordinateur selon l'une quelconque des revendications 11 ou 12, dans lequel le programme thérapeutique comprend un premier programme thérapeutique et un second programme thérapeutique, et dans lequel l'indication de la valeur d'intervalle de recharge suggérée comporte une première indication pour le premier programme thérapeutique et une seconde indication pour le second programme thérapeutique, la première indication étant différente de la seconde indication.

14. Support de stockage non transitoire lisible par ordinateur selon l'une quelconque revendication 13, dans lequel le procédé comprend en outre :
la sortie, par la circuiterie de traitement, d'une indication d'une transition entre le premier programme thérapeutique et le second programme thérapeutique.
